# EUROPEAN PATENT APPLICATION

(11) **EP 4 353 144 A1**
(43) Date of publication of application: **17.04.2024**
(21) Application number: 22883871.0
(22) Date of filing: 13.10.2022
(51) Int. Cl.: A61B 5/00, A61B 5/01, A61B 5/024, A61B 5/1455, A61B 5/0205

(54) **WEARABLE ELECTRONIC DEVICE COMPRISING BIOSENSORS FOR MEASURING BODY TEMPERATURE**

(30) Priority: 18.10.2021 KR 20210138744; 19.11.2021 KR 20210160677; 31.08.2022 KR 20220109664
(71) Applicant: Samsung Electronics Co., Ltd., Suwon-si, Gyeonggi-do 16677 (KR)
(72) Inventor: KIM, Kijung, Suwon-si Gyeonggi-do 16677 (KR); KANG, Junghyun, Suwon-si Gyeonggi-do 16677 (KR); LEE, June, Suwon-si Gyeonggi-do 16677 (KR); CHOI, Jaejoon, Suwon-si Gyeonggi-do 16677 (KR)
(74) Representative: Gulde & Partner
(86) International application number: PCT/KR2022/015472
(87) International publication number: WO 2023/068648

(57) **Abstract**

An exemplary wearable electronic device may comprise: a housing having a first hole formed therein and comprising a rear surface cover coming into contact with at least a part of a user's body when the wearable electronic device is worn by the user; a first biosensor disposed inside the housing and aligned to the first hole, the first biosensor comprising a first lens disposed in the first hole and set to sense first biometric data of the user on the basis of a predetermined wavelength band light passing through the first lens; a second biosensor disposed inside the housing and facing a rear surface cover, the second biosensor set to sense second biometric data which is different from the first biometric data; and a shielding structure for preventing foreign materials from entering through the first hole.

## Description

### [Technical Field]

The disclosure relates to an electronic device including a biometric sensor for measuring a body temperature.

### [Background Art]

In recent years, not only hand-held type electronic devices, such as smartphones and tablet personal computers (PCs), which may be carried by user, but also wearable type electronic devices, such as smart watches, smart glasses, and earbuds, which may be mounted on bodies of users, have been actively developed.

Moreover, demands for wearable electronic devices equipped with healthcare functions have increased as interests in health have been increased among people.

One healthcare function is to provide body temperature information of a user. To provide body temperature information to a user, a wearable electronic device may include a temperature sensor. Temperature sensors for measuring a body temperature may be classified into contact type temperature sensors and noncontact type temperature sensors.

### [Disclosure]

### [Technical Problem

Because a contact type temperature sensor essentially has to contact a body of a user, an error of a measured temperature value may be generated due to a habit of a user who wears the electronic device, a mounting portion, and the like, and some users may have resistances against the temperature sensors that directly contact the skin.

Meanwhile, the noncontact type sensor may measure a body temperature while not directly contacting the skin. A sensor using an infrared ray is an example of a noncontact type sensor. The infrared ray temperature sensor uses infrared ray energy of a specific wavelength band, which is emitted by an object having an absolute temperature of 0 K or more through convection, for example, with infrared energy in a wavelength band of 8 µm to 14 µm, which is a long wave infrared (LWIR) band. Because an intensity of infrared ray energy is radiated according to a temperature of an object, the infrared ray temperature sensor may detect an intensity of infrared ray energy radiated from a body of a person and convert the detected intensity into a temperature, and may provide body temperature information to a user.

Meanwhile, the infrared ray temperature sensor may require a lens that transmits an infrared ray to detect the intensity of the infrared ray energy. For example, a lens having an infrared ray transmissivity of a specific level or more (e.g., a transmissivity of about 70% or more) may be required for an operation of the infrared ray temperature sensor. When hardness is low, even though transmissivity may be satisfied, the lens may be broken and thus a user may be injured. When a glass material of a relatively high hardness is used for the lens, it is difficult to satisfy a transmissivity of a specific wavelength required for measurement of temperature.

Embodiments of the disclosure may provide a wearable electronic device including a biometric sensor for measuring a body temperature, which satisfies a safety of a user, transmissivity characteristics required for measurement of temperature, and/or a waterproof performance.

### [Technical Solution]

A wearable electronic device according to various example embodiments of the disclosure includes a housing including a rear cover having a first hole and configured to at least partially contact a body of a user while the wearable electronic device is worn by the user, a first biometric sensor disposed in the housing and aligned with the first hole, wherein the first biometric sensor includes a first lens disposed in the first hole and is configured to detect first biometric information of the user based on light of a designated wavelength band, which passes through the first lens, a second biometric sensor disposed in the housing and disposed to face the rear cover, wherein the second biometric sensor is configured to detect second biometric information that is distinguished from the first biometric information, and a blocking structure configured to block (e.g., reduce) foreign substances from being introduced through the first hole.

A wearable electronic device according to an embodiment of the disclosure includes a frame, a display disposed on one side of the frame, a rear cover disposed on an opposite side of the frame, and having a first seating hole, a second seating hole, and a third seating hole, a printed circuit board disposed between the display and the rear cover, a processor disposed in the printed circuit board, and a biometric sensor module disposed between the printed circuit board and the rear cover, and including a first biometric sensor and a second biometric sensor operatively connected to the processor, wherein the second biometric sensor includes a first light emitting part (e.g., emitter), a second light emitting part, and a plurality of light receiving parts (e.g., receivers) that overlap the rear cover, a first lens disposed in the first seating hole, and at least partially overlapping the first biometric sensor, a second lens disposed in the second seating hole, and at least partially overlapping the first light emitting part of the second biometric sensor, and a third lens disposed in the third seating hole, and at least partially overlapping the second light emitting part of the second biometric sensor, and the processor configured to detect a body temperature of a user as first biometric information, based on light of a designated wavelength band, which is received using the first biometric sensor, and to detect second biometric information that is distinguished from the first biometric information, using the second biometric sensor.

### [Advantageous Effects]

According to various example embodiments of the disclosure, a wearable electronic device including a biometric sensor for measuring a body temperature, to which a foreign substance blocking structure is applied, may be provided.

According to various example embodiments, a lens that satisfies a transmissivity of a wavelength band, which is advantageous for measuring a body temperature of a biometric sensor, may be provided.

According to various example embodiments, through a plurality of fine holes formed in the lens and the optic member filled therein, a light transmissivity of a wavelength band, which is advantageous for measuring a body temperature by a biometric sensor, may be enhanced while mechanical characteristics and/or waterproof/dustproof performances are maintained.

In addition, various example embodiments of the disclosure may provide various effects that are directly or indirectly recognized.

### [Description of Drawings]

The above and other aspects, features and advantages of certain embodiments of the present disclosure will be more apparent from the following detailed description, taken in conjunction with the accompanying drawings, in which:
FIG. 1 is a perspective view of a front surface of an example mobile electronic device according to various embodiments;
FIG. 2 is a perspective view of a rear surface of an example electronic device according to various embodiments;
FIG. 3 is an exploded perspective view of an example electronic device according to various embodiments;
FIG. 4A is an exploded perspective view illustrating certain configurations of an example electronic device according to various embodiments;
FIG. 4B is a view of a rear cover of an example electronic device according to various embodiments.
FIG. 4C is a cross-sectional view, taken along line A-A' of FIG. 4B;
FIG. 4D is a partially enlarged view illustrating area A1 of FIG. 4C;
FIG. 5A illustrates an example electronic device including a first waterproof structure according to various embodiments;
FIG. 5B illustrates an example electronic device including a second waterproof structure according to various embodiments;
FIG. 5C illustrates an example electronic device including a third waterproof structure according to various embodiments;
FIGS. 6A and 6B illustrate examples of lenses according to various embodiments;
FIG. 7A is an exploded perspective view of an example electronic device according to various embodiments;
FIG. 7B is a view illustrating an example biometric sensor module according to various embodiments;
FIG. 7C is a view of a rear cover of an example electronic device according to various embodiments;
FIG. 7D is a cross-sectional view, taken along line C-C' of FIG. 7C;
FIG. 8A is a view illustrating an example first lens according to various embodiments;
FIG. 8B is a view illustrating an example first lens according to various embodiments;
FIG. 9 illustrates an example electronic device in a network environment according to various embodiments;
FIG. 10A is a view illustrating an example electronic device according to various embodiments;
FIG. 10B is a view illustrating an example electronic device according to various embodiments;
FIG. 11A is a cross-sectional view illustrating an example electronic device according to various embodiments;
FIG. 11B is a cross-sectional view illustrating an example electronic device according to various embodiments; and
FIG. 12 illustrates an example electronic device according to various embodiments.

With regard to description of drawings, the same or similar components may be marked by the same or similar reference numerals.

### [Mode for Invention]

Hereinafter, various example embodiments of the disclosure will be described with reference to the accompanying drawings. Accordingly, those of ordinary skill in the art will recognize that modifications, equivalents, and/or alternatives of the various embodiments described herein can be variously made without departing from the scope and spirit of the disclosure.

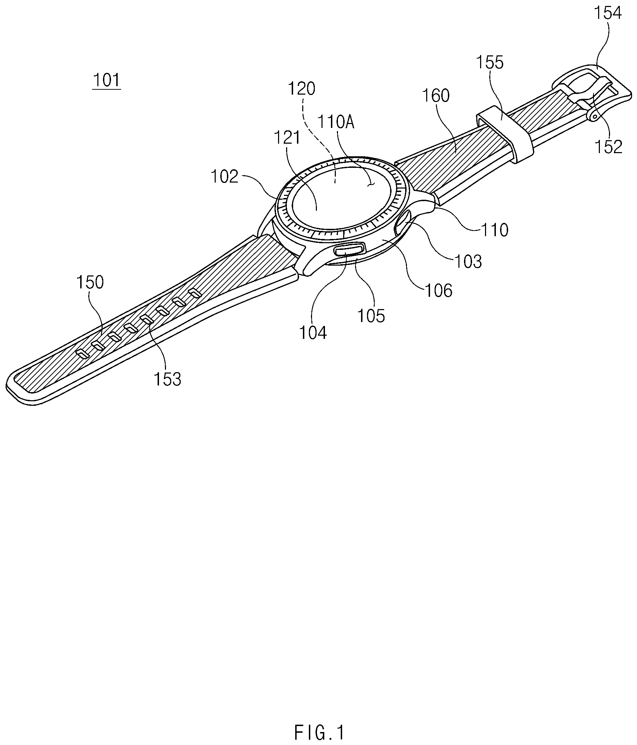

FIG. 1 is a perspective view of a front surface of an example mobile electronic device according to various embodiments.

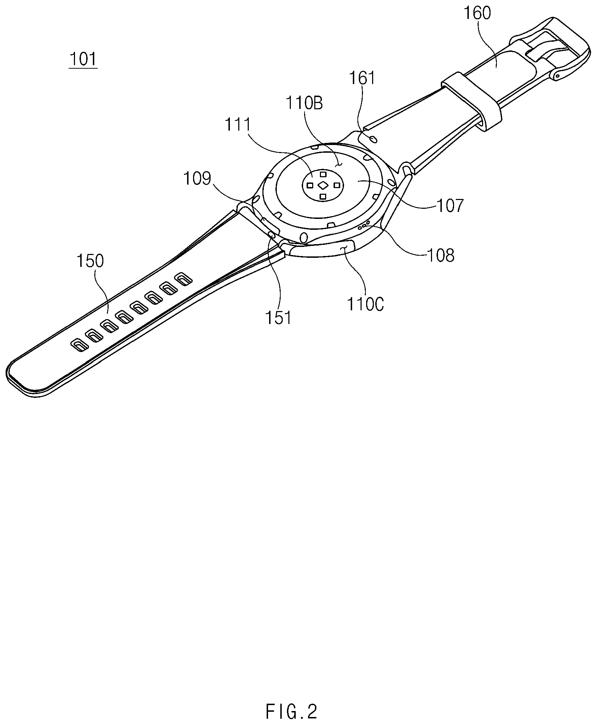

FIG. 2 is a perspective view of a rear surface of an example electronic device according to various embodiments.

Referring to FIGS. 1 and 2, an electronic device 101 according to an embodiment may include a housing 110 including a first surface (or a front surface) 110A, a second surface (or a rear surface) 110B, and a side surface 110C surrounding a space between the first surface 110A and the second surface 110B, and fastening members 150 and 160 connected to at least portions of the housing 110 and that detachably fastens the electronic device 101 to a portion (e.g., a wrist or an ankle) of the body of a user. In an embodiment (not illustrated), the housing 110 may refer to a structure that forms some of the first surface 110A, the second surface 110B, and the side surface 110C of FIG. 1.

According to an embodiment, the first surface 110A may be defined by a front plate 121 (e.g., a glass plate or a polymer plate including various coating layers), at least a portion of which is substantially transparent. The second surface 110B may be defined by a substantially opaque rear plate 107. The rear plate 107, for example, may be formed of coated or colored glass, ceramics, a polymer, a metal (e.g., aluminum, stainless steel (STS), or magnesium), or a combination of at least two thereof. The side surface 110C may be coupled to the front plate 121 and the rear plate 107, and may be defined by a side bezel structure (or 'a side member') 106 including a metal and/or a polymer. In various embodiments, the rear plate 107 and the side bezel structure 106 may be integrally formed and may include the same material (e.g., a metallic material such as aluminum).

The fastening members 150 and 160 may be formed of various material and have various shapes. A single body or a plurality of unit links that may move with respect to each other may be formed of woven fabric, leather, rubber, urethane, a metal, ceramics, or a combination of at least two thereof.

According to an embodiment, the electronic device 101 may include at least one of a display 120, audio modules 105 and 108, a sensor module 111, key input devices 102, 103, and 104, and a connector hole 109. In some embodiments, at least one (e.g., the key input devices 102, 103, and 104, the connector hole 109, or the sensor module 111) may be omitted from the electronic device 101 or another component may be additionally included in the electronic device 101.

The display 120, for example, may be visually exposed through the front plate 121. The shape of the display 120 may correspond to the shape of the front plate 121, and may include various shapes, such as a circular shape, an elliptical shape, or a polygonal shape. The display 120 may be coupled to or be disposed to be adjacent to a touch detection circuit, a pressure sensor that may measure an intensity (a pressure) of a touch, and/or a fingerprint sensor.

The audio modules 105 and 108 may include the microphone hole 105 and the speaker hole 108. A microphone for acquiring external sounds may be disposed in the microphone hole 105, and in some embodiments, a plurality of microphones may be disposed to detect the direction of a sound. The speaker hole 108 may be used for an external speaker and a communication receiver. In various embodiments, the speaker hole 108 and the microphone hole 105 may be realized by one hole or a speaker may be included in which the speaker hole 108 is not employed (e.g., a piezoelectric speaker).

The sensor module 111 (e.g., including one or more sensors) may generate an electrical signal or a data value corresponding to an operation state of the interior of the electronic device 101 or an (external) environmental state of the outside. The sensor module 111, for example, may include a Heart Rate Monitor (HRM) sensor disposed on the second surface 110B of the housing 110. The sensor module may further include , for example and without limitation, at least one of a gesture sensor, a gyro sensor, an atmospheric pressure sensor, a magnetic sensor, an acceleration sensor, a grip sensor, a color sensor, an infrared (IR) sensor, a biometric sensor, a temperature sensor, a humidity sensor, or an illumination sensor.

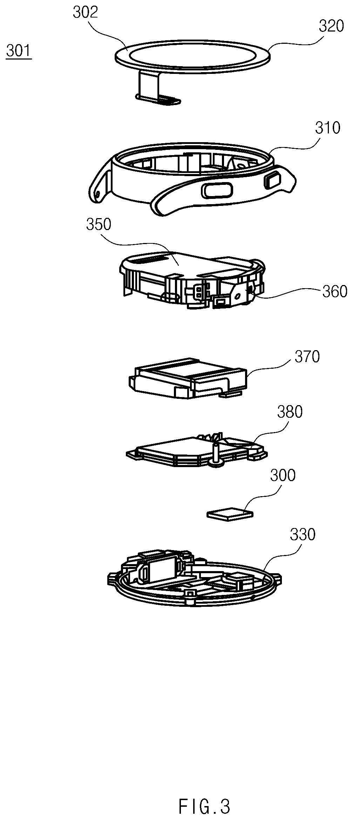

The key input devices 102, 103, and 104 may include a wheel key 102 disposed on the first surface 110A of the housing 110 and which is rotatable in at least one direction, and/or the side key buttons 103 and 104 disposed on the side surface 110C of the housing 110. The wheel key 102 may have a shape corresponding to the shape of the front plate 121. In an embodiment, the electronic device 101 may not include some or all of the above-mentioned key input devices 102, 103, and 104, and other key input devices may be realized in different forms, such as a soft key, on the display 120. For example, the wheel key 102 may be implemented in a form, in which it is operated based on a touch signal input to the display 120 (e.g., a wheel key area 302 of FIG. 3).

The connector hole 109 may accommodate a connector (e.g., a USB connector) for transmitting and receiving power and/or data to and from an external electronic device, and may include another connector hole (not illustrated) that may accommodate a connector for transmitting and receiving an audio signal to and from an external electronic device. The electronic device 101, for example, may further include a connector cover (not illustrated) that covers at least a portion of the connector hole 109 to prevent or reduce introduction of external foreign substances through the connector hole 109.

The fastening members 150 and 160 may be detachably fastened to at least a partial area of the housing 110 using locking members 151 and 161. The fastening members 150 and 160 may include one or more of a fixing member 152, a fixing member coupling hole 153, a band guide member 154, and a band fixing ring 155.

The fixing member 152 may be configured to fix the housing 110 and the fastening members 150 and 160 to a portion (e.g., a wrist or an ankle) of the body of the user. The fixing member coupling hole 153 may fix the housing 110 and the fastening members 150 and 160 to a portion of the body of the user in correspondence to the fixing member 152. The band guide member 154 may be configured to restrict a motion range of the fixing member 152 when the fixing member 152 is coupled to the fixing member coupling hole 153 so that the fastening members 150 and 160 are fastened to be attached to a portion of the body of the user. The band fixing ring 155 may restrict a motion range of the fastening members 150 and 160 in a state in which the fixing member 152 and the fixing member coupling hole 153 are coupled to each other.

FIG. 3 is an exploded perspective view of an example electronic device according to various embodiments. Referring to FIG. 3, an electronic device 301 (e.g., the electronic device 101 of FIG. 1) may include a display 320, a frame 310, an antenna 350, a support member 360 (e.g., a bracket), a battery 370, a printed circuit board 380 (e.g., a printed circuit board (PCB), a printed board assembly (PBA), a flexible PCB (FPCB) or a rigid-flexible PCB (RFPCB)), a rear cover 330, and a biometric sensor module 300 (e.g., including at least one biometric sensor). At least one of the components of the electronic device 301 may be the same as or similar to at least one of the components of the electronic device 101 of FIGS. 1 and 2, and a description thereof will not be repeated here. The electronic device 301 may include at least one of the elements of the electronic device 101 of FIGS. 1 and 2. For example, the electronic device 301 may include the fastening members 150 and 160, which are not illustrated in FIG. 3.

In an embodiment, the display 320 (e.g., the display 120 of FIG. 1) may be at least partially accommodated in the frame 310. The display 320 may include the wheel key area 302 formed at a periphery thereof. The wheel key area 302 may be configured to receive a touch input of a user. In an embodiment, the electronic device 301 may perform a function that is substantially the same as a function performed through the wheel key 102 of FIG. 1, based on a touch input received through the wheel key area 302. In this case, the physically implemented wheel key 102 may be omitted.

In an embodiment, the frame 310 (e.g., the side bezel structure 106 of FIG. 1) may define an external appearance (e.g., the side surface 110C of FIG. 1) of the electronic device 301. In an embodiment, the frame 310 may provide a space, in which various elements of the electronic device 301 are disposed or accommodated. For example, the display 320 may be disposed on one side of the frame 310, and the rear cover 330 may be coupled to an opposite side thereof. The antenna 350, the support member 360, the battery 370, the printed circuit board 380, and the biometric sensor module 300 may be disposed in an interior of a space defined by the frame 310, the display 320, and the rear cover 330. In an embodiment, at least a portion of the display 320 (or the front plate 121 of FIG. 1), the frame 310, and/or the rear cover 330 may be referred to, for example, as a housing (e.g., the housing 110 of FIG. 1) of the electronic device 301 in that it defines an external appearance of the electronic device 301 and provides a space, in which various elements of the electronic device 301 are accommodated.

In an embodiment, the support member 360 may be disposed in an interior of the electronic device 301 to be connected to the frame 310, or may be integrally formed with the frame 310. The support member 360, for example, may be formed of a metallic material and/or a nonmetallic material (e.g., a polymer). The display 320 may be coupled to one surface of the support member 360, and the printed circuit board 380 may be coupled to an opposite surface of the support member 360.

In an embodiment, a processor (e.g., including processing circuitry), a memory, and/or an interface (e.g., including interface circuity) may be disposed in the printed circuit board 380. The processor, for example, may include one or more of a central processing unit, an application processor, a graphic processing unit (GPU), an application processor, a sensor processor, or a communication processor. The memory, for example, may include a volatile and/or nonvolatile memory. The interface, for example, may include a high definition multimedia interface (HDMI), a universal serial bus (USB) interface, an SD card interface, and/or an audio interface. The interface, for example, may electrically or physically connect the electronic device 301 to an external electronic device, and may include a USB connector, an SD card/MMC connector, and an audio connector.

In an embodiment, the battery 370 supplies electric power to at least one component of the electronic device 301, and for example, may include a secondary battery that may be recharged. The battery 370 may be integrally disposed in the interior of the electronic device 301, or may be disposed to be detachable from the electronic device 301.

In an embodiment, the antenna 350 may be disposed between the display 320 and the support member 360. The antenna 350, for example, may include a near field communication (NFC) antenna, a wireless charging antenna, and/or a magnetic secure transmission (MST) antenna. The antenna 350, for example, may perform short-range communication with an external device, may wirelessly transmit and receive electric power that is necessary for charging, and may transmit a short range communication signal or a magnetic-based signal (e.g., including payment data).

In an embodiment, the housing of the electronic device 301 may at least partially define an antenna structure for wireless communication. For example, the antenna structure may be formed by or include a portion or a combination of the frame 310 and/or the support member 360. In an embodiment, the antenna structure may be formed by a portion or a combination of the frame 310 and/or the rear cover 330. In an embodiment, the electronic device 301, for example, may perform cellular communication and short-range wireless communication, such as Wi-Fi, or Bluetooth, or receive a global positioning system (GPS) signal, through the antenna structure.

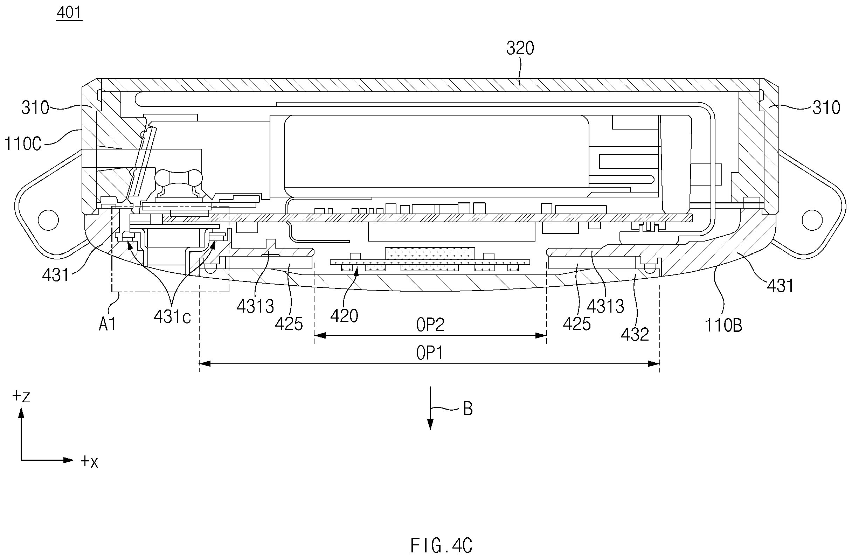

The electronic device 301 according to an embodiment, although not illustrated, may include a wireless charging coil (e.g., a wireless charging module 425 of FIG. 4C) that transmits and receives an electric power signal to and from an external device.

In an embodiment, the biometric sensor module 300 may be disposed between the printed circuit board 380 and the rear cover 330. In an embodiment, the biometric sensor module 300 may at least partially face the rear cover 330. For example, the biometric sensor module 300 may be disposed to face the rear cover 330 that at least partially contacts a wrist of the user while the user wears the electronic device 301. In an embodiment, the biometric sensor module 300 may include at least one sensor that acquires the biometric information (e.g., a heartbeat rate, an oxygen saturation, a body temperature) of the user.

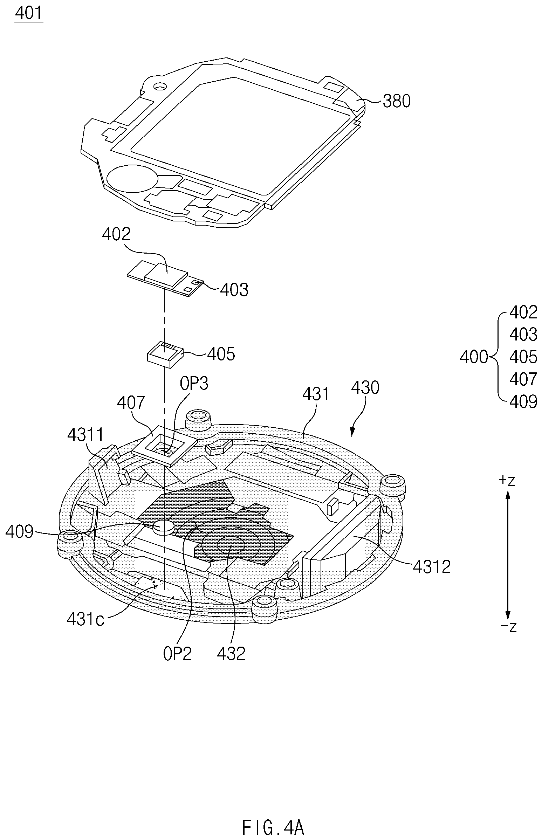

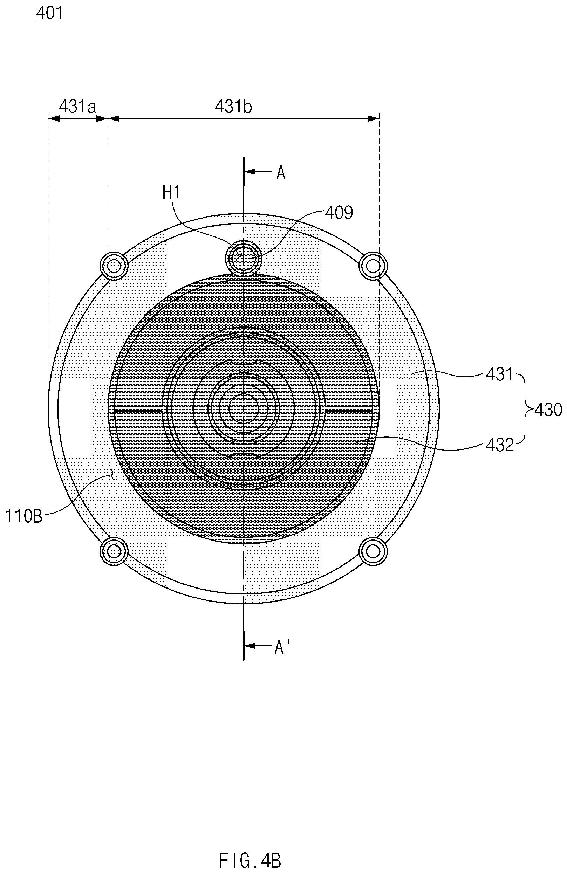

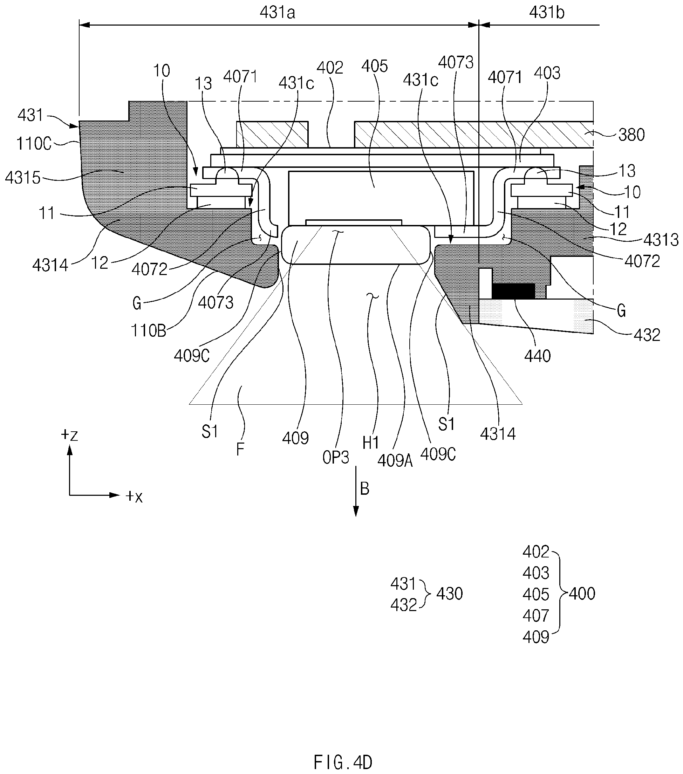

FIG. 4A is an exploded perspective view illustrating certain configurations of an example electronic device according to various embodiments. FIG. 4B is a view of a rear cover of an example electronic device according to various embodiments. FIG. 4C is a cross-sectional view, taken along line A-A' of FIG. 4B. FIG. 4D is a partially enlarged view illustrating an area A1 of FIG. 4C. Direction "B" illustrated in FIGS. 4C and 4D may, for example, be a direction that faces the body of the user while an electronic device 401 is mounted on (being worn by) the user. Hereinafter, a description of configurations having the same reference numerals as the above-described configurations will not be repeated.

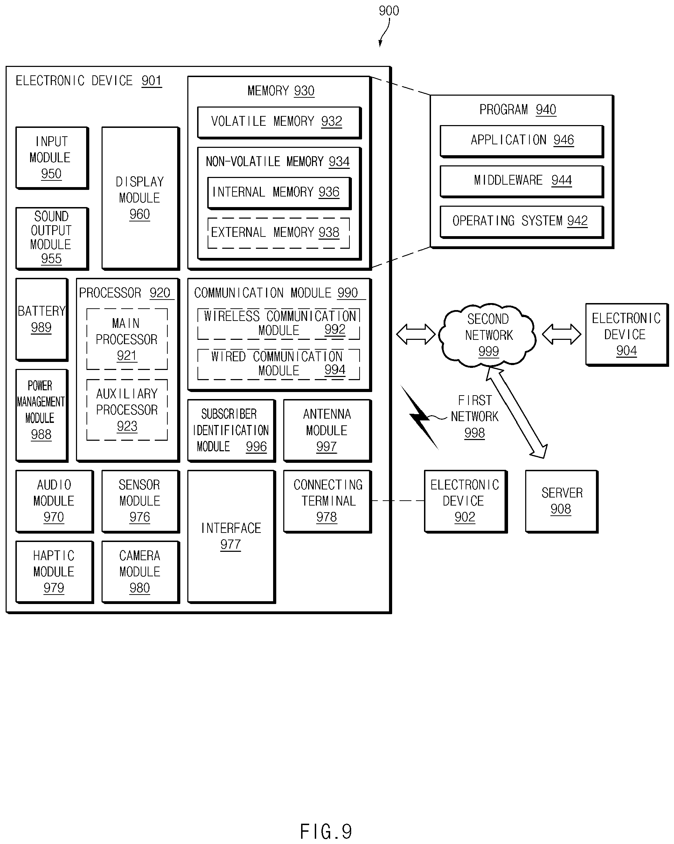

Referring to FIGS. 4A, 4B, 4C, and 4D, a rear cover 430 (e.g., the rear cover 330 of FIG. 3) of the electronic device 401 (e.g., the electronic device 301 of FIG. 3) according to various embodiments may include a first member 431 and a second member 432. In an embodiment, the first member 431 may include a first connection part 4311 and a second connection part 4312 that extend from an outer peripheral part thereof toward the display 320. The first connection part 4311 and the second connection part 4312 may be located on an inner side of the frame 310. The first connection part 4311 and/or the second connection part 4312 may be connected to the frame 310. One or more other elements, for example, a speaker (e.g., a sound output module 955 of FIG. 9) of the electronic device 401 may be disposed in the first connection part 4311 and/or the second connection part 4312, but the disclosure is not limited in this respect. In an embodiment, the second member 432 of the rear cover 430 may be disposed under the first member 431 (e.g., the -z axis direction) to be coupled to the first member 431. A second opening OP2 may be formed at a central portion of the first member 431. The second opening OP2 may be closed by the second member 432. In an embodiment, the first member 431 may include a resin or ceramic. In an embodiment, the second member 432 may include glass. For example, the second member 432 may include glass formed of or including a silica-based material, such as borosilicate glass or aluminosilicate glass. As another example, the second member 432 may be formed of or include an alumina-based material, such as corundum. For example, the second member 432 may include sapphire crystal or sapphire glass. However, the disclosure is not limited by the above-described examples.

As illustrated in FIG. 4B, the first member 431 of the rear cover 430 may include an outer part 431a that is located outside the second member 432 to be exposed to an outside, and an inner part 431b that overlaps the second member 432 not to be exposed to the outside due to the second member 432. In an embodiment, a first hole H1 may be formed in the first member 431, and the first hole H1 may be located in the outer part 431a of the first member 431. In an embodiment, the first hole H1 may extend from the rear surface 110B of the first member 431 to a seating part 431c, and may pass through the first member 431.

In an embodiment, the electronic device 401 may include a first biometric sensor 400 (e.g., the biometric sensor module 300 of FIG. 3). The first biometric sensor 400 may be disposed between the printed circuit board 380 and the rear cover 430. For example, the first biometric sensor 400 may be disposed between the printed circuit board 380 and the first member 431 of the rear cover 430. The first biometric sensor 400 may be at least partially disposed (or accommodated) in the seating part 431c provided in the first member 431 of the rear cover 430. In an embodiment, the seating part 431c may include at least a portion of an inner surface of the first member 431, which faces an opposite direction to the rear surface 110B and a first inner peripheral surface S1 of the first hole H1, in which the first biometric sensor 400 is disposed. The first biometric sensor 400 may be partially accommodated in the first hole H1. The first biometric sensor 400 may be (externally) exposed to an outside through the first hole H1. In an embodiment, the rear surface 110B of the rear cover 430 may at least partially contact the body of the user while the electronic device 401 is mounted on (being worn by) the user. In an embodiment, the first biometric sensor 400 may acquire the biometric information of the user through the first hole H1.

In an embodiment, the first biometric sensor 400 may include a board 403, an elastic member 402, a sensor part 405 (including a sensor), a sensor housing 407, and a lens 409. In an embodiment, the board 403 may include a printed circuit formed of a conductive material (e.g., copper). The board 403 may include a flexible printed circuit board (FPCB) that may be at least partially bent, but the disclosure is not limited in this respect.

In an embodiment, the board 403 may be electrically connected to the printed circuit board 380. For example, the board 403 may be electrically connected to the printed circuit board 380 through a conductive pad(s) provided on one surface thereof and a connection member (e.g., a C-clip) that contacts the conductive pad(s). As another example, a plug may be provided at one end of the board 403, and the board 403 and the printed circuit board 380 may be electrically connected to each other in a scheme, in which the plug is coupled to a receptacle provided in the printed circuit board 380. As another example, the board 403 may be electrically and/or physically joined to the printed circuit board 380 through a hot-bar scheme. However, the above-described examples are not limited, and various methods may be used.

In an embodiment, the elastic member 402 and the sensor part 405 may be disposed in the board 403. For example, the elastic member 402 may be disposed on one surface (e.g., a surface that faces the printed circuit board 380) of the board 403, and the sensor part 405 may be disposed on an opposite surface (a surface that faces the lens 409) to the one surface. The elastic member 402 may be located between the board 403 and the printed circuit board 380, and the sensor part 405 may be located between the board 403 and the rear cover 430. In an embodiment, the sensor part 405 may be at least partially aligned with the first hole H1 of the rear cover 430. For example, the sensor part 405 may at least partially overlap the first hole H1. The sensor part 405 may be disposed to face the lens 409 disposed in the first hole H1 such that a light receiving part (not illustrated) (e.g., including a light receiver) included in the sensor part 405 receives light through the first hole H1 and the lens 409.

In an embodiment, the sensor housing 407 may be disposed in the board 403 to at least partially surround the sensor part 405. The sensor housing 407 may include a metal. For example, the sensor housing 407 may include phosphor bronze or stainless steel, but the disclosure is not limited in this respect. For example, various kinds of metals may be applied as the material of the sensor housing 407 in consideration of a machining property, a corrosion property, and a strength that is required in the first biometric sensor 400.

In an embodiment, the lens 409 may be coupled to the sensor housing 407. For example, the lens 409 may be coupled to the sensor housing 407 in a scheme in which it is at least partially inserted into a light receiving hole OP3 formed in the sensor housing 407. The lens 409 may at least partially overlap the sensor part 405. For example, the lens 409 may at least partially overlap the sensor part 405 with respect to the z axis.

In an embodiment, the lens 409 may be at least partially disposed in the first hole H1. The lens 409 may be at least partially exposed to the outside of the electronic device 401 through the first hole H1 of the rear cover 430. The light outside the electronic device 401 may be input to the lens 409 through the first hole H1. In an embodiment, the lens 409 may be configured to transmit light of a designated wavelength band. For example, the lens 409 may be configured to transmit an infrared ray of an LWIR band. For example, the lens 409 may be formed of a silicon material (e.g., silicon lens) to transmit the light of the LWIR band. As another example, the lens 409 may include glass including zinc or diamond, which may transmit the light of the LWIR band. However, the disclosure is not limited by the above-described example. The light that passes through the lens 409 may reach the sensor part 405.

In an embodiment, the sensor part 405 may include an inner housing that defines an external appearance thereof, at least one sensor (or at least one light receiving part) disposed in the inner housing, and a control circuit (or a controller integrated circuit (IC) or a driver integrated circuit (IC)) for an operation of the first biometric sensor 400. The control circuit may be electrically connected to the at least one sensor. In an embodiment, the sensor part 405 may be operatively connected to another element of the electronic device 401 disposed in the printed circuit board 380 through an electric path provided by the board 403 and the printed circuit board 380. For example, the first biometric sensor 400 may be operatively connected to a processor (e.g., a processor 920 of FIG. 9) of the electronic device 401. For example, the control circuit of the sensor part 405 may be electrically connected to the processor.

In an embodiment, the at least one sensor of the sensor part 405 may be disposed to face the lens 409 to receive the infrared ray that passes through the lens 409. The at least one sensor may output a sensing signal (an electric power signal corresponding to an intensity of the received infrared ray) based on the received light. The control circuit may process the sensing signal output from the at least one sensor. For example, the control circuit may process the sensing signal and provide a biometric signal (or data) including temperature information corresponding to the sensing signal to the processor (e.g., the processor 920 of FIG. 9). The processor may acquire first biometric information that represents temperature, based on the provided biometric signal. The first biometric information may be a temperature obtained through conversion, based on infrared ray energy that is radiated from the body of the user and is detected, that is, a body temperature of the user. However, a process of acquiring the biometric information corresponding to the light detected by using the sensor part 405 is not limited to the above-described examples, and various methods may be used.

Referring to FIGS. 4C and 4D, the first member 431 according to an embodiment may have a first opening OP1 and the second opening OP2. The first opening OP1 and/or the second opening OP2, which communicate with each other, may pass through the first member 431. For example, the first opening OP1 may be formed on the rear surface 110B of the first member 431 to extend toward the second opening OP2, and the second opening OP2 may extend from the first opening OP1 to an inner surface (e.g., a surface that is opposite to the rear surface 110B of the first member 431) of the first member 431. The first member 431 may include a support part 4313. In an embodiment, the diameter of the first opening OP1 may be formed to be larger than that of the second opening OP2 to define the support part 4313.

The electronic device 401 according to an embodiment may include a second biometric sensor 420. The second biometric sensor 420 (e.g., the sensor module 111 of FIG. 2) may be disposed between the printed circuit board 380 and the second member 432 of the rear cover 430. The second biometric sensor 420 may be at least partially disposed in the first opening OP1 and the second opening OP2 formed at a central portion of the first member 431.

In an embodiment, the second biometric sensor 420 may be disposed to face the second member 432. For example, the second biometric sensor 420 may be disposed to face direction "B" on the second member 432 to acquire the second biometric information on the body of the user located in direction B while the user wears the electronic device 401.

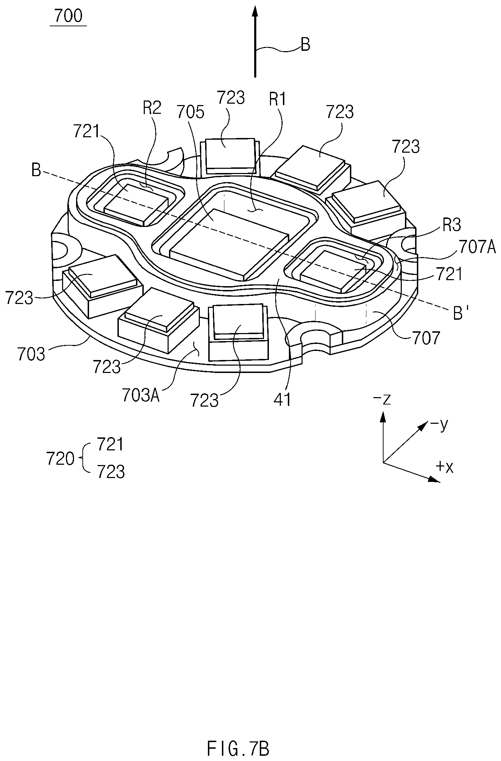

In an embodiment, the second biometric sensor 420 may be configured to acquire the second biometric information that is different from the first biometric information acquired using the first biometric sensor 400. For example, the second biometric sensor 420 may include at least one light emitting part (e.g., first light emitting parts 721 of FIG. 7B), and at least one light receiving part (e.g., first light receiving parts 723 of FIG. 7B). The at least one light emitting part, for example, may include a light emitting diode (LED) or a laser diode (LD). The at least one light receiving part, for example, may include a photodiode or an image sensor. The second biometric sensor 420 may detect the light that is input to the light receiving part(s) after the light emitted from the light emitting part(s) is reflected by the body of the user to acquire the second biometric information. The second biometric information, for example, may include information on the heartbeat rate of the user and/or information on oxygen saturation. The second biometric sensor 420 may measure a heartbeat rate by detecting a reflectivity (or transmissivity) of light according to a change in an amount of blood in blood vessels according to contraction/relaxation of the heart (a change in a volume of the blood vessel according to the amount of blood). The second biometric sensor 420 may measure an oxygen saturation by detecting a reflectivity (or transmissivity) of the light according to an amount of oxygen in the blood.

In an embodiment, a range of wavelengths of the light emitted by the second biometric sensor 420, for example, may be about 380 nm to about 800 nm. In an embodiment, the second member 432 of the rear cover 430 may be formed of a material (e.g., glass) that may transmit the light emitted from the second biometric sensor 420 and the light received by the second biometric sensor 420.

The electronic device 401 according to an embodiment may include the wireless charging module 425 (e.g., including wireless charging circuitry). The wireless charging module 425, for example, may include a flat plate type coil. The wireless charging module 425 may generate an electric current using electromagnetic induction generated by an external electronic device (e.g., a wireless charging pad). The electronic device 401 may charge a battery (e.g., the battery 370 of FIG. 3) using the electric current generated by the wireless charging module 425.

In an embodiment, the wireless charging module 425 may be disposed between the support part 4313 of the first member 431 and the second member 432. The wireless charging module 425 may have a shape that at least partially surrounds a periphery of the second biometric sensor 420.

In an embodiment, the lens 409 may include a first surface 409A that faces direction B, and a side surface 409C that extends from a periphery of the first surface 409A toward the board 403. In an embodiment, the lens 409 may be at least partially located in the first hole H1 and the light receiving hole OP3. For example, the lens 409 may be disposed such that the side surface 409C thereof is adjacent to a third part 4073 of the sensor housing 407, which defines the light receiving hole OP3 and the first inner peripheral surface S1 of the first hole H1.

In an embodiment, the sensor housing 407 may include a first part 4071, a second part 4072, and the third part 4073. The first part 4071 may be seated on the board 403. The first part 4071 may extend to be substantially parallel to the board 403. When the viewed from a top of the board (e.g., when viewed in the z axis direction), the first part 4071 may have a shape that surrounds the sensor part 405. The second part 4072 may extend from the first part 4071. For example, the second part 4072 may extend from an inner periphery of the first part 4071 in a direction that becomes farther from the board 403. In an embodiment, the second part 4072 may extend along a surface (e.g., the inner housing of the sensor part 405) of the sensor part 405. In an embodiment, the second part 4072 may extend along a surface of the sensor part 405. As another example, the second part 4072 may extend from the first part 4071 along the first inner peripheral surface S1 of the first hole H1. As another example, the second part 4072 may extend from the first part 4071 toward the rear surface 110B. The second part 4072 may extend from the inner periphery of the first part 4071 to the third part 4073. When the viewed from the top of the board 403 (e.g., when viewed in the z axis direction), the second part 4072 may have a shape that surrounds the sensor part 405. The third part 4073 may extend from the second part 4072 toward the lens 409. The light receiving hole OP3, into which the lens 409 is at least partially inserted, may be formed in the third part 4073. The third part 4073 may at least partially contact the side surface 409C of the lens 409. When the viewed from the top of the board 403 (e.g., when viewed in the z axis direction), the third part 4073 may have a shape that surrounds the side surface 409C of the lens 409. Because the third part 4073 at least partially covers the side surface 409C of the lens 409 that is vulnerable to a physical impact, damage to the lens 409 may be reduced or prevented.

In an embodiment, the first member 431 of the rear cover 430 may include the support part 4313, a bottom part 4314, and a wall part 4315. The support part 4313 may have the second opening OP2, in which the second biometric sensor 420 is partially disposed. The support part 4313 may support the wireless charging module 425, and the second member 432 of the rear cover 430. The bottom part 4314 may extend from the support part 4313 toward an outward direction (e.g., the side surface 110C) of the rear cover 430, to the wall part 4315. The bottom part 4314 may protrude in a downward direction (e.g., the -z axis direction) of the support part 4313 to define the first opening OP1. The first opening OP 1 may have a diameter that is larger than that of the second opening OP2. The second member 432 of the rear cover 430 may be disposed in the first opening OP1. The second member 432 may be seated on the support part 4313 to close the first opening OP1 and the second opening OP2. In an embodiment, a sealing member 440 (e.g., including a seal) may be disposed between the second member 432 and the support part 4313 to reduce or prevent foreign substances from being introduced between the first member 431 and the second member 432 of the rear cover 430. The wall part 4315 may extend from an outer periphery of the bottom part 4314 in a height direction (e.g., the +z axis direction) of the electronic device 401. For example, the wall part 4315 may extend from an outer periphery of the bottom part 4314 toward the frame 310. The wall part 4315 may define the side surface 110C of the electronic device 401 together with the frame 310.

In an embodiment, the first member 431 of the rear cover 430 may include the inner part 431b that overlaps the second member 432, and the outer part 431a that does not overlap the second member 432 and is exposed to the outside. The inner part 431b may include the support part 4313 of the first member 431. The outer part 431a may include the bottom part 4314 and the wall part 4315 of the first member 431. The first hole H1 may be formed in one area of the outer part 43 1a. The first hole H1 may pass through the bottom part 4314. For example, the first hole H1 may permit communication between the rear surface 110B corresponding to the bottom part 4314 and a surface of the bottom part 4314, which is opposite to the rear surface 110B.

In an embodiment, the first biometric sensor 400 may be disposed in the bottom part 4314 of the first member 431. For example, the first biometric sensor 400 may be disposed on the seating part 431c formed on the surface of the bottom part 4314, which faces the opposite direction to the rear surface 110B. The seating part 431c may have a shape corresponding to a shape of the first biometric sensor 400. For example, the seating part 431c may include at least a portion of the surface of the bottom part 4314, on which the first part 4071 of the sensor housing 407 is seated, and the first inner peripheral surface S1 of the first hole H1, in which the sensor housing 407 and the lens 409 are at least partially accommodated.

In an embodiment, the first hole H1 may be formed to have one or more different diameters according to the shape of the first biometric sensor 400 inserted thereinto. Furthermore, the first hole H1 may be formed to not overlap a viewing angle "F" of the first biometric sensor 400. For example, the bottom part 4314 that defines the first hole H1 may include a chamfered portion not to overlap the viewing angle "F", and the first inner peripheral surface S1 of the first hole H1 may include an area that extends while being inclined. For example, the first hole H1 may be formed such that a diameter thereof increases as it becomes closer to the rear surface 110B (e.g., as it goes in the -z axis direction).

Referring to FIG. 4D, in an embodiment, the electronic device 401 may include a first waterproof structure 10. The first waterproof structure 10 may include a first waterproof member 11 and an adhesion member 12. The first waterproof member 11 may be disposed between the first part 4071 of the sensor housing 407 and the adhesion member 12. The first waterproof member 11 may have a shape that surrounds the second part 4072 of the sensor housing 407. The first waterproof member 11 may include a boss 13 that protrudes toward the sensor housing 407, and the boss 13 may be pressed by the sensor housing 407. For example, the boss 13 may protrude toward the first part 4071 of the sensor housing 407 and be pressed by the first part 4071. The first waterproof member 11, for example, may include rubber. The adhesion member 12 may be disposed between the first waterproof member 11 and the bottom part 43 14. The adhesion member 12 may have a shape that is substantially the same as or substantially similar to the first waterproof member 11, and may bond the first waterproof member 11 to the bottom part 4314. The adhesion member 12, for example, may include a double-sided tape. In an embodiment, because moisture may be introduced between the first waterproof member 11 and the bottom part 4314 when a width (e.g., a thickness of the adhesion member 12 with respect to the x axis) of the adhesion member 12 is too small, the width of the adhesion member 12 may be determined within a range, by which a waterproof performance of the first waterproof structure 10 may be maintained.

In an embodiment, unlike the illustration of FIG. 4D, the first waterproof member 11 may be disposed in a bottom part 4314, and the adhesion member 12 may be disposed between the first waterproof member 11 and the sensor housing 407. In this case, the boss 13 of the first waterproof member 11 may protrude toward the bottom part 4314 to be pressed by the bottom part 4314.

In an embodiment, the first waterproof structure 10 may prevent moisture from being introduced through a gap "G" formed as the first biometric sensor 400 is spaced apart from the first inner peripheral surface S1 of the first hole H1.

In an embodiment, the elastic member 402 may be configured to provide a force in a direction that faces the rear cover 430 to the first biometric sensor 400 in a state, in which the first biometric sensor 400 and the printed circuit board 380 are assembled. For example, the elastic member 402 may include rubber, but the disclosure is not limited thereto. In an embodiment, the elastic member 402 located between the board 403 and the printed circuit board 380 may provide an elastic force in a direction that faces the lens 409 (or the rear cover 430) to the board 403. The elastic force provided by the elastic member 402 may be delivered to the first waterproof structure 10 through the board 403 and the sensor housing 407. Through this, a waterproof performance of the first waterproof structure 10 may be enhanced. For example, when the elastic member 402 is interposed between the board 403 and the printed circuit board 380, the first waterproof structure 10 may maintain the waterproof performance even at a higher pressure.

Although not illustrated, one or more waterproof members may, for example, be formed between the sensor part 405 and the sensor housing 407, between the sensor part 405 and the lens 409, and/or between the lens 409 and the sensor housing 407. The waterproof member may be formed by applying a resin solution having a waterproof property and an adhesion property to the sensor housing 407 before assembling, assembling the sensor part 405 and the lens 409, and curing the applied solution. Through the waterproof member, foreign substances, such as moisture and dust, may be reduced or prevented from being introduced into an interior of the first biometric sensor 400. The resin solution, for example, may include an epoxy-based resin, but the disclosure is not limited in this respect.

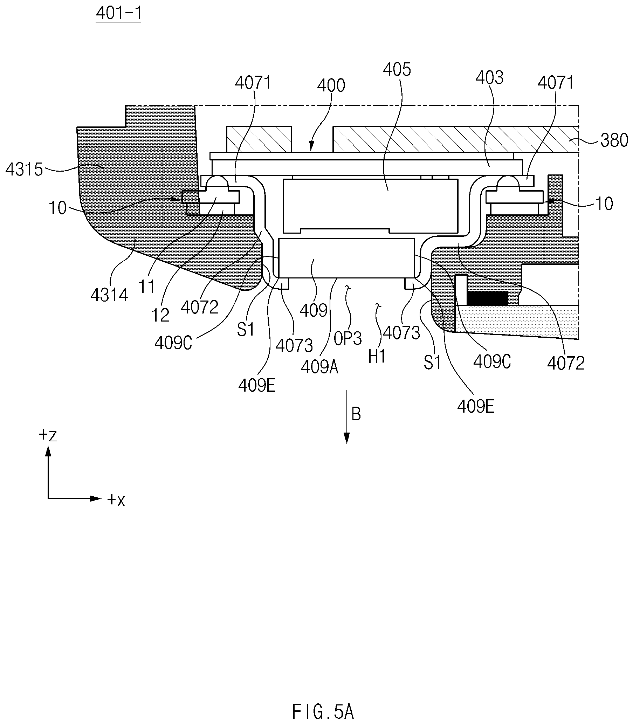

FIG. 5A illustrates an example electronic device including a first waterproof structure according to various embodiments. FIG. 5A is a view illustrating a part of an example electronic device according to various embodiments, which corresponds to the area A1 of FIG. 4C.

Referring to FIG. 5A, the sensor housing 407 of an electronic device 401-1 according to various embodiments may surround the side surface 409C and the first surface 409A of the lens 409. For example, the third part 4073 of the sensor housing 407 may extend to a peripheral area of the first surface 409A over the side surface 409C of the lens 409. The third part 4073 of the sensor housing 407 may surround the entire side surface 409C of the lens 409, and may cover the peripheral area of the first surface 409A. In an embodiment, the sensor housing 407 may cover an edge part 409E that is a border of the first surface 409A and the side surface 409C of the lens 409. The sensor housing 407 may prevent or reduce damage to the lens 409 by protecting the edge part 409E of the lens 409 that is vulnerable to a physical impact.

In an embodiment, the light receiving hole OP3 that is defined by the third part 4073 of the sensor housing 407 may be formed to be smaller than a diameter of the lens 409. The light receiving hole OP3 may be located under the lens 409 (e.g., direction B). When the board 403 is viewed from the top (e.g., when viewed in the z axis direction), the third part 4073 of the sensor housing 407 may partially overlap the lens 409. For example, the third part 4073 may overlap a peripheral area of the first surface 409A of the lens 409. In an embodiment, the third part 4073 of the sensor housing 407 may prevent or reduce the lens 409 from being moved in direction B or being separated from the sensor housing 407.

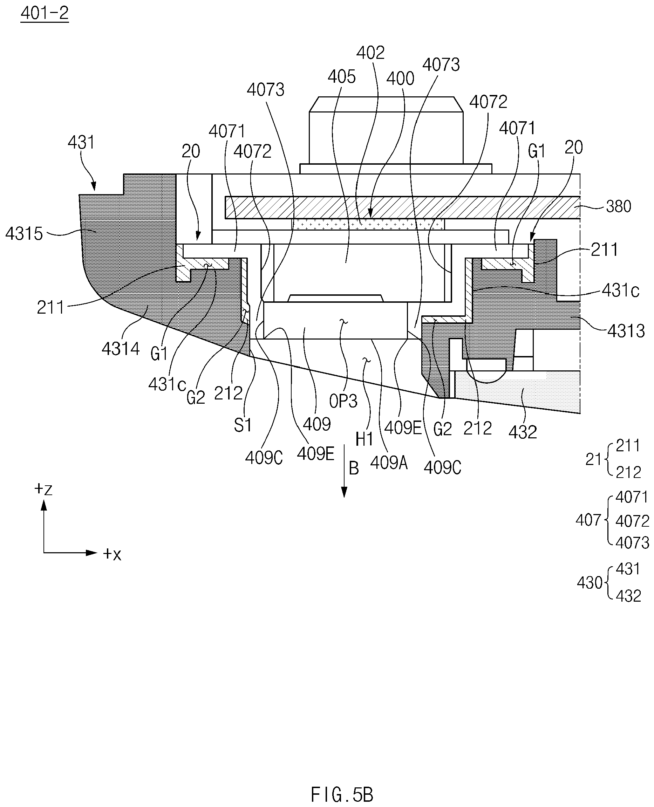

In an embodiment, the second part 4072 and the third part 4073 of the sensor housing 407 may be adjacent to the first inner peripheral surface S1 of the first hole H1. A waterproof adhesion layer (not illustrated) (e.g., the waterproof adhesion layer 21 of FIG. 5B) formed by applying and curing a bonding liquid may be formed between the first inner peripheral surface S1 of the first hole H1 and the sensor housing 407, but the disclosure is not limited in this respect. The waterproof adhesion layer may at least partially fill in the gap "G" illustrated in FIG. 4D. In various embodiments, the waterproof adhesion layer may be viewed as being included in the first waterproof structure 10.

In an embodiment, the first waterproof member 11 of the first waterproof structure 10 may be pressed by the wall part 4315. The first waterproof member 11 may be pressed by both of the first part 4071 of the sensor housing 407 and the wall part 4315 of the first member 431. Because the first waterproof member 11 is pressed by the wall part 4315 of the first member 431 and the sensor housing 407, foreign substances may be prevented or reduced from being introduced more effectively than in a case, in which only the boss 13 of the first waterproof member 11 is pressed by the sensor housing 407.

FIG. 5B illustrates an example electronic device including a second waterproof structure according to various embodiments. FIG. 5B is a view illustrating a part of an electronic device according to various embodiments, which corresponds to area A1 of FIG. 4C.

Referring to FIG. 5B, an electronic device 401-2 according to another embodiment may include a second waterproof structure 20. The second waterproof structure 20 may include the waterproof adhesion layer 21. The waterproof adhesion layer 21 may be interposed between the sensor housing 407 and the rear cover 430. The waterproof adhesion layer 21 may include a first layer 211 and a second layer 212. The first layer 211 may be interposed between the first part 4071 of the sensor housing 407 and the bottom part 4314 of the first member 431. The first layer 211 may at least partially fill in a first gap G1. The first gap G1 may be formed between the first part 4071 of the sensor housing 407 and the bottom part 4314 (and/or the wall part 4315) of the first member 431. The second layer 212 may be interposed between the second part 4072 (and/or the third part 4073) of the sensor housing 407 and the first inner peripheral surface S1 of the first hole H1. The second layer 212 may at least partially fill in a second gap G2. The second gap G2 may be formed between the second part 4072 (and/or the third part 4073) of the sensor housing 407 and the first inner peripheral surface S1 of the first hole H1 . In an embodiment, the waterproof adhesion layer 21, for example, may be formed as a bonding solution including a resin is cured after being applied. The first layer 211 may include parts, thicknesses of which are different according to a shape of the first gap G1, but the disclosure is not limited in this respect. The second layer 212 may include parts, thicknesses of which are different according to a shape of the second gap G2, but the disclosure is not limited in this respect.

In FIG. 5B, the third part 4073 of the sensor housing 407 covers both of the side surface 409C and the edge part 409E of the lens 409, but may not cover the first surface 409A.

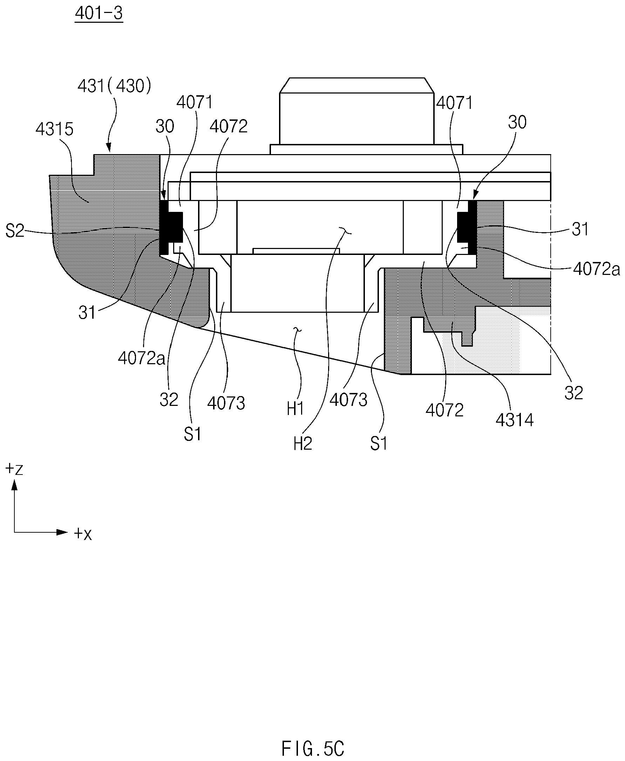

FIG. 5C illustrates an example electronic device including a third waterproof structure according to various embodiments. FIG. 5C is a view illustrating a part of an electronic device according to various embodiment, which corresponds to area A1 of FIG. 4C.

Referring to FIG. 5C, the sensor housing 407 of the electronic device 401-3 according to another embodiment ma include a protrusion 4072a. The protrusion 4072a may extend from the second part 4072 of the sensor housing 407 in a direction that becomes farther from the sensor part 405. The protrusion 4072a may be spaced apart from the first part 4071. When viewed from a top (e.g., when viewed in the z axis direction), the protrusion 4072a may at least partially overlap the first part 4071. For example, the protrusion 4072a may extend from the second part 4072 to face the first part 4071. As another example, the protrusion 4072a may extend in a direction that is substantially perpendicular to an extension direction of the second part 4072, but the disclosure is not limited in this respect.

In an embodiment, the first hole H1 and a second hole H2 may be formed in the first member 431 of the rear cover 430. The second hole H2 may communicate with the first hole H1, and may have a diameter that is larger than that of the first hole H1. When the board 403 is viewed from the top (e.g., when viewed in the z axis direction), the first inner peripheral surface S1 of the first hole H1 may be located in a second inner peripheral surface S2 of the second hole H2. The first biometric sensor 400 may be at least partially accommodated in the first hole H1 and the second hole H2.

In an embodiment, the electronic device 401-3 may include a third waterproof structure 30. The third waterproof structure 30 may include a second waterproof member 31. The second waterproof member 31 may be disposed between the wall part 4315 of the first member 431 and the sensor housing 407. For example, the second waterproof member 31 may be disposed at least between the second inner peripheral surface S2 of the second hole H2 and the sensor housing 407. The second waterproof member 31 may be interposed between a recess defined by the first part 4071, the second part 4072, and the protrusion 4072a of the sensor housing 407, and the second inner peripheral surface S2 of the second hole H2 to be compressed by the sensor housing 407. The second waterproof member 31, for example, may include a sealing member such as an O-ring formed of or including a rubber material.

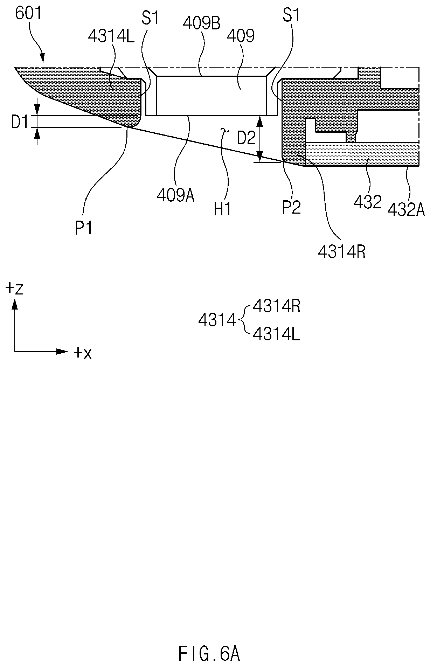

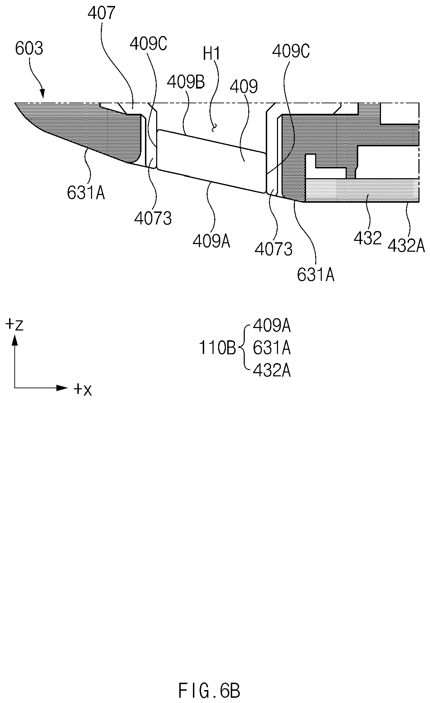

FIGS. 6A and 6B illustrate examples of lenses according to various embodiments. Referring to FIGS. 6A and 6B, the lens 409 may include the first surface 409A facing direction B (e.g., a direction facing the body of the user wearing the electronic device), and a second surface 409B that faces an opposite direction to the first surface 409A. Although FIGS. 6A and 6B illustrates that the second surface 409B is substantially parallel to the first surface 409A, the disclosure is not limited to the illustrated examples. A relative inclination, curvatures, or shapes of the first surface 409A and the second surface 409B of the lens 409 may be changed according to optical characteristics that are required or desirable in the lens 409, and various design changes are possible.

Referring to reference numeral 601 of FIG. 6A, the lens 409 may not protrude to an outside of the first hole H1. For example, the lens 409 may be inserted into an interior of the first member 431 of the rear cover 430. The lens 409 may be disposed in an interior of the first hole H1 such that the first surface 409A is located above the rear surface 110B (e.g., the z axis direction).

In an embodiment, the first surface 409A of the lens 409 may be spaced apart from a first border P1 of the rear surface 110B corresponding to a first member 4314L located on the left side with respect to the illustration of reference numeral 601 in FIG. 6A, and the first inner peripheral surface S1, by a first distance D1. In an embodiment, the first surface 409A of the lens 409 may be spaced apart from a second border P2 of the rear surface 110B corresponding to a first member 4314R located on the right side with respect to the illustration of reference numeral 601 in FIG. 6A, and the first inner peripheral surface S 1, by a second distance D2. Because the rear surface 110B of the rear cover 430 is at least partially curved and the lens 409 is disposed substantially perpendicularly to the first hole H1 formed in a vertical direction, the first distance D1 and the second distance D2 may be different. Furthermore, the second distance D2 may be larger than the first distance D1. In an embodiment, because the lens 409 is disposed to be inserted into the interior of the rear cover 430, the lens 409 may be protected from a physical impact. For example, when the electronic device drops, the first border P1 or the second border P2 collides earlier than the lens 409, and thus an impact applied to the lens 409 may be reduced.

Referring to reference numeral 603 of FIG. 6B, in an embodiment, the first surface 409A of the lens 409 may define a surface that is substantially the same as the rear surface 110B. For example, the first surface 409A of the lens 409 may extend with no step from the rear surface 110B defined by the first member 431 (or substantially seamlessly). In this case, the rear surface 110B may be defined by a first rear surface 631A of the first member 431, a second rear surface 432A of the second member 432, and the first surface 409A of the lens 409. In an embodiment, because the sensor housing 407 extends to the first surface 409A to cover the side surface 409C of the lens 409, it may be understood that the sensor housing 407 also defines the rear surface 110B of the electronic device together.

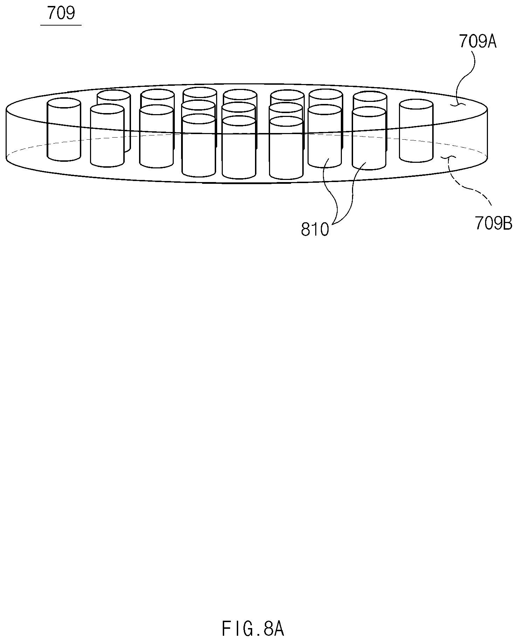

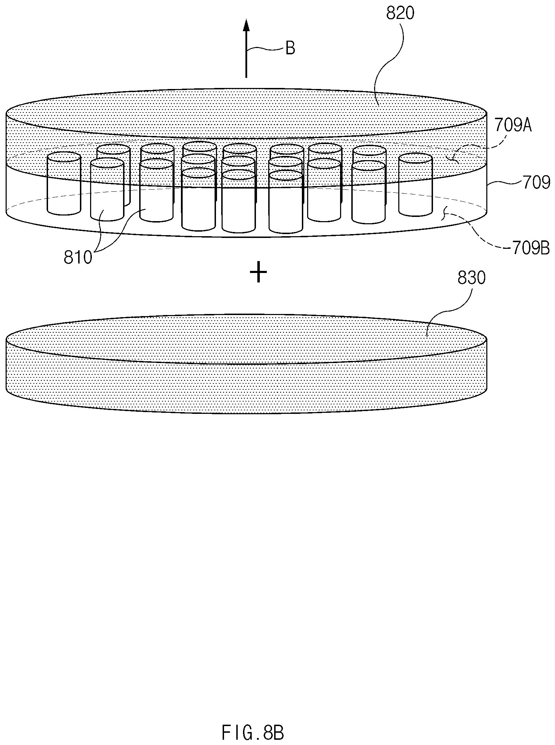

Although not illustrated, a description made below with reference to a first lens 709 may be applied to the lens 409 alternatively or additionally. For example, the lens 409 may include a plurality of holes 810, which will be described with reference to FIG. 8A, and optic members filled therein. As another example, a first protection layer 820 and/or a second protection layer 830, which will be described with reference to FIG. 8B, may be disposed on the first surface 409A and/or the second surface 409B of the lens 409. As another example, the lens 409 may include a fourth waterproof structure or a fifth waterproof structure, which will be described with reference to FIGS. 8A and 8B.

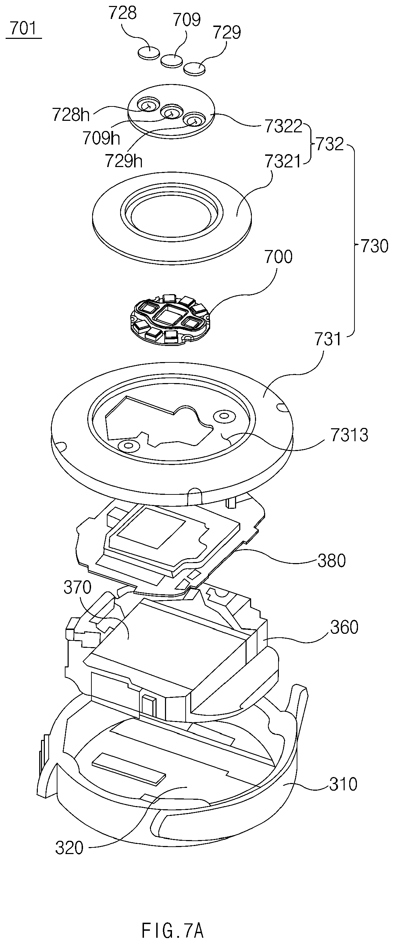

FIG. 7A is an exploded perspective view of an example electronic device according to various embodiments. Referring to FIG. 7A, an electronic device 701 (e.g., the electronic device 301 of FIG. 3 or the electronic device 401 of FIG. 4A) according to various embodiments may include a rear cover 730, the first lens 709, a second lens 728, a third lens 729, and a biometric sensor module 700.

In an embodiment, the rear cover 730 may include a first member 731 and a second member 732. In an embodiment, the first member 731 may correspond to the first member 431 illustrated in FIGS. 4C and 4D. For example, the description made with reference to the first member 431 may be applied to the first member 731 in a substantially same, similar, or corresponding manner.

In an embodiment, the second member 732 may include a first sub-member 7321 and a second sub-member 7322. The first sub-member 7321 and the second sub-member 7322 may be coupled to each other. For example, the first sub-member 7321 and the second sub-member 7322 may be coupled to each other in a form, in which the second sub-member 7322 closes a hollow formed in the first sub-member 7321. The first sub-member 7321 may be coupled to the first member 731. For example, the first sub-member 7321 may be seated on a support part 7313 (e.g., the support part 4313 of FIG. 4D) of the first member 731 to be supported by the first member 731. In an embodiment, the second sub-member 7322 may include glass. For example, the second sub-member 7322 may include glass formed of or including a silica-based material, such as borosilicate glass or aluminosilicate glass. As another example, the second sub-member 7322 may be formed of or include an alumina-based material, such as corundum. For example, the second sub-member 7322 may include sapphire crystal or sapphire glass. However, the disclosure is not limited by the above-described examples. The first sub-member 7321 may include glass, a resin, or ceramic. In an embodiment, the first sub-member 7321 may be formed of a material that is substantially the same as or different from that of the second sub-member 7322. In an embodiment, when the first sub-member 7321 is formed of a material (e.g., glass) that is the same as that of the second sub-member 7322, the first sub-member 7321 and the second sub-member 7322 may be integrally formed, but the disclosure is not limited in this respect.

In an embodiment, the first lens 709, the second lens 728, and the third lens 729 may be seated on a first seating hole 709h, a second seating hole 728h, and a third seating hole 729h formed in the second sub-member 7322, respectively.

In an embodiment, the biometric sensor module 700 (e.g., the biometric sensor module 300 of FIG. 3) may be disposed between the rear cover 730 and the printed circuit board 380. The biometric sensor module 700 may be disposed such that a plurality of sensors included in the biometric sensor module 700 face the rear cover 730. For example, the biometric sensor module 700 may be disposed such that the plurality of sensors face the second sub-member 7322 of the rear cover 730. The biometric sensor module 700 may at least partially overlap the second sub-member 7322. In an embodiment, the biometric sensor module 700 may perform a function that is substantially the same as that of the first biometric sensor (e.g., the first biometric sensor 400 of FIG. 4A) and the second biometric sensor (e.g., the second biometric sensor 420 of FIG. 4C). For example, in an embodiment, the electronic device 701 may acquire the first biometric information (e.g., a body temperature) and the second biometric information (e.g., a heartbeat rate or an oxygen saturation) using the biometric sensor module 700.

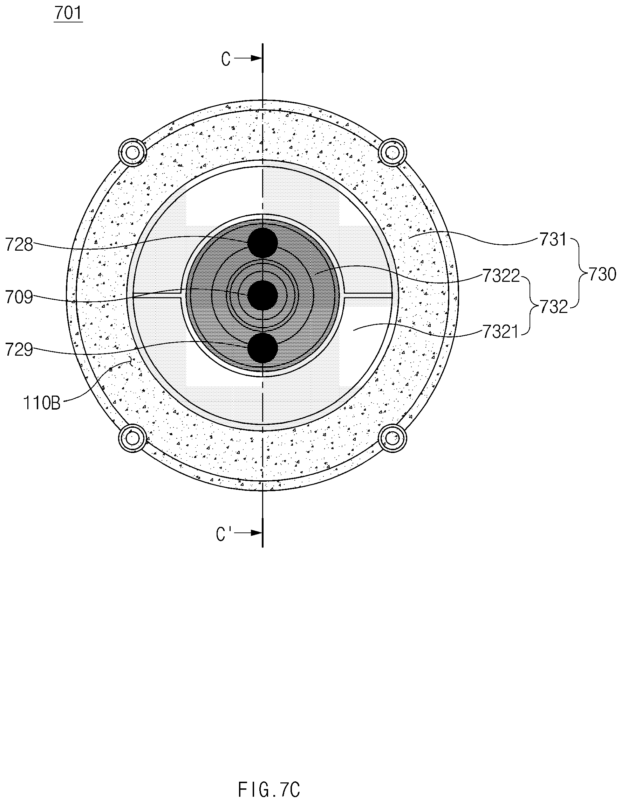

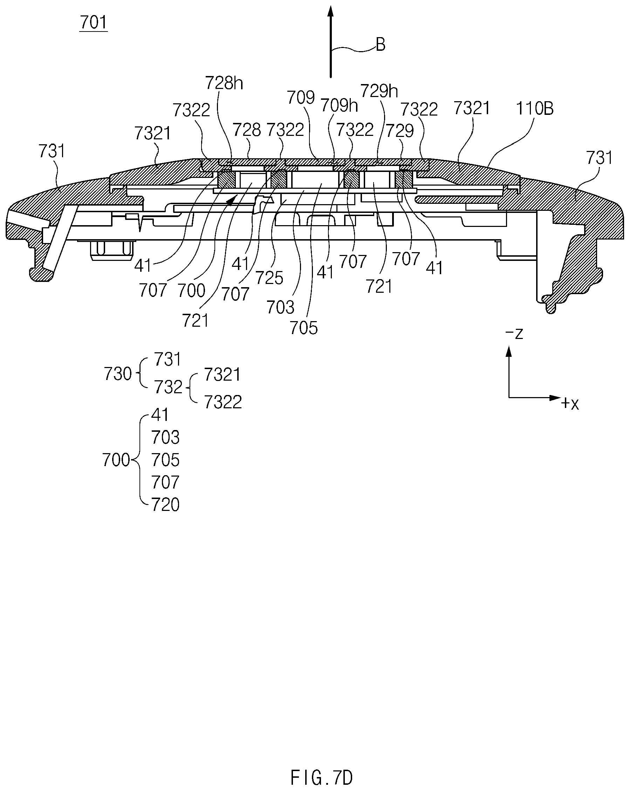

FIG. 7B is a view illustrating an example biometric sensor module according to various embodiments. Line B-B' of FIG. 7B may correspond to line C-C' of FIG. 7C. FIG. 7C is a view of the rear cover of an example electronic device according to various embodiments. FIG. 7D is a cross-sectional view, taken along line C-C' of FIG. 7C. Direction "B" illustrated in FIGS. 7B and 7D may be a direction that faces the rear cover (e.g., the rear cover 730 of FIG. 7A) from the biometric sensor module 700.

Referring to FIGS. 7B, 7C, and 7D, in an embodiment, the biometric sensor module 700 may include a board 703, a partition wall structure 707, a first biometric sensor 705, a second biometric sensor 720, and a light shielding member 41.

In an embodiment, the board 703 (e.g., the board 403 of FIG. 4A) may include one surface 703A that faces direction "B". The partition wall structure 707, the first biometric sensor 705, and the second biometric sensor 720 may be disposed on the one surface 703A of the board 703. Although not illustrated, the board 703 may include a connector for connecting the board 703 to the printed circuit board 380 of the electronic device 701.

In an embodiment, the partition wall structure 707 may extend from the one surface 703A of the board 703 in the height direction (e.g., -z axis direction) of the electronic device 701. For example, the partition wall structure 707 may extend from the one surface 703A of the board 703 toward the rear cover 730. The partition wall structure 707 may define a first recess R1, a second recess R2, and a third recess R3, together with the one surface 703A of the board 703. The first recess R1, the second recess R2, and the third recess R3 may be separated by the partition wall structure 707. The first recess R1, the second recess R2, and the third recess R3 may be at least partially arranged on the board 703 along one direction (e.g. the x axis direction). The first recess R1 may be located between the second recess R2 and the third recess R3.

In an embodiment, the partition wall structure 707 may be formed of a magnet. In this case, the partition wall structure 707 may correspond to a magnet provided in an external electronic device (e.g., a wireless charging pad) (not illustrated) when electric power is received from the external electronic device using a wireless charging coil (e.g., the wireless charging module 425 of FIG. 4C). Because the partition wall structure 707 corresponds to the magnet provided in the external electronic device, the electronic device 701 may be stably held in the external electronic device, and the wireless charging module may be arranged in place. Through this, the partition wall structure 707 may allow a stable wireless charging operation of the electronic device 701. In an embodiment, the partition wall structure 707 may be formed of a resin instead of a magnet.

In an embodiment, the first biometric sensor 705 may be disposed in the first recess R1. In an embodiment, the first biometric sensor 705 may correspond to the first biometric sensor 400 (or the sensor part 405) of FIGS. 4A and 4B. For example, the first biometric sensor 705 may include at least one sensor (or a light receiving part) that is configured to detect an infrared ray (or infrared ray energy) radiated from the body of the user. The first biometric sensor 705 may acquire first biometric information (e.g., a body temperature) of the user, based on the infrared ray detected using the at least one sensor.

In an embodiment, the second biometric sensor 720 may include first light emitting parts 721 and first light receiving parts 723. In an embodiment, the first light emitting parts 721 may be disposed in the second recess R2 and the third recess R3 located on opposite sides of the first recess R1. In an embodiment, the first light receiving parts 723 may be disposed on the one surface 703A of the board 703, which corresponds to an outer area of the partition wall structure 707. For example, some of the first light receiving parts 723 may be disposed on one side (e.g., the +y axis direction) with respect to the partition wall structure 707, and the remaining ones may be disposed on an opposite side (e.g., the -y axis direction) with respect to the partition wall structure 707.

In an embodiment, the second biometric sensor 720 may correspond to the second biometric sensor 420 of FIG. 4C. For example, the second biometric sensor 720 may emit light in direction "B" using the first light emitting parts 721. The emitted light may be reflected by the body of the user and may reach the first light receiving parts 723. The second biometric sensor 720 may acquire the second biometric information (e.g., a heartbeat rate and/or an oxygen saturation) using the first light emitting parts 721 and the first light receiving parts 723.

In an embodiment, by the partition wall structure 707, the first biometric sensor 705, and the first light emitting parts 721 and the first light receiving parts 723 of the second biometric sensor 720 may be separated. The partition wall structure 707 may prevent or reduce the light emitted from the first light emitting parts 721 from failing to be reflected by the body of the user and then reaching the first light receiving parts 723 and the first biometric sensor 705. For example, the partition wall structure 707 may prevent the light emitted from the first light emitting parts 721 from being directly input to the first biometric sensor 705 and the first light receiving parts 723, by surrounding the first light emitting parts 721. In an embodiment, the partition wall structure 707 may interrupt light that is emitted from the first light emitting parts 721 and is reflected from a location other than the body of the user (e.g., light that fails to pass through the second lens 728 after being emitted from the first light emitting parts 721 and then is reflected) from reaching the first light receiving parts 723 and the first biometric sensor 705, by surrounding the first light receiving parts 723 and the first biometric sensor 705. Furthermore, the partition wall structure 707 may interrupt interference light (e.g., light other than the light that is reflected by the body of the user and then reaches the first light receiving parts 723) from reaching the first light receiving parts 723. Furthermore, the partition wall structure 707 may interrupt interference light (e.g., light other than the infrared ray (or infrared ray energy) radiated from the body of the user) from reaching the first biometric sensor 705.

In an embodiment, the light shielding member 41 may be disposed on the partition wall structure 707. For example, the light shielding member 41 may be disposed on an upper surface 707A, in which the partition wall structure 707 faces direction "B". When the board 703 is viewed from the top (e.g., when viewed in the z axis direction), the light shielding member 41 may have a shape that is substantially the same as or substantially similar to that of the partition wall structure 707. In an embodiment, the light shielding member 41 disposed on the partition wall structure 707 may include a material that may be compressed by the rear cover 730. For example, the light shielding member 41 may include sponge or rubber. Additionally or alternatively, the light shielding member 41 may include a material that may attach the rear cover 730 to the partition wall structure 707 while being compressed by the rear cover 730. For example, the light shielding member 41 may include a double-sided tape. When the partition wall structure 707 is formed rigidly, an aperture may be formed between the partition wall structure 707 and the rear cover 730 disposed on the partition wall structure 707. Through the aperture, light associated with the first biometric sensor 705 and the second biometric sensor 720 may leak. In an embodiment, the light shielding member 41 may be compressed by the rear cover 730 to seal the partition wall structure 707 and the rear cover 730, and may prevent or reduce the light associated with the first biometric sensor 705 and the second biometric sensor 720 from being interfered.

In an embodiment, the first biometric sensor 705 may correspond to the first lens 709 disposed in the first seating hole 709h of the second sub-member 7322. For example, the first biometric sensor 705 may be at least partially aligned with the first seating hole 709h of the second sub-member 7322 and the first lens 709 disposed therein. For example, the first biometric sensor 705 may at least partially overlap the first lens 709. The first biometric sensor 705 may detect the first biometric information through the light that passes through the first lens 709 to be input. For example, the infrared ray radiated from the body of the user may pass through the first lens 709 and may reach the first biometric sensor 705. The first biometric sensor 705 may detect the input infrared ray, and may sense the body temperature of the user based on the detected infrared ray.

In an embodiment, the first light emitting parts 721 of the second biometric sensor 720 may correspond to the second lens 728 and the third lens 729 disposed in the second seating hole 728h and the third seating hole 729h of the second sub-member 7322, respectively. For example, among the first light emitting parts 721, the light emitting part disposed in the second recess R2 may at least partially overlap the second lens 728, and the light emitting part disposed in the third recess R3 may at least partially overlap the third lens 729. The light emitted from the first light emitting parts 721 may pass through the second lens 728 and the third lens 729, and may reach the body of the user.

In an embodiment, the first light receiving parts 723 of the second biometric sensor 720 may correspond to the second sub-member 7322. For example, the first light receiving parts 723 of the second biometric sensor 720 may at least partially overlap the second sub-member 7322. In an embodiment, the light that is emitted from the first light emitting parts 721 and reflected by the body of the user may pass through the second sub-member 7322 and may reach the first light receiving parts 723.

In an embodiment, the first lens 709, the second lens 728, and the third lens 729 may define the rear surface 110B of the electronic device 701 together with the rear cover 730. For example, the first lens 709, the second lens 728, and the third lens 729 may define a surface that is substantially the same as that of the rear cover 730. For example, the surfaces of the first lens 709, the second lens 728, and the third lens 729 may extend with no step from the rear surface 110B of the rear cover 730 (or substantially seamlessly).

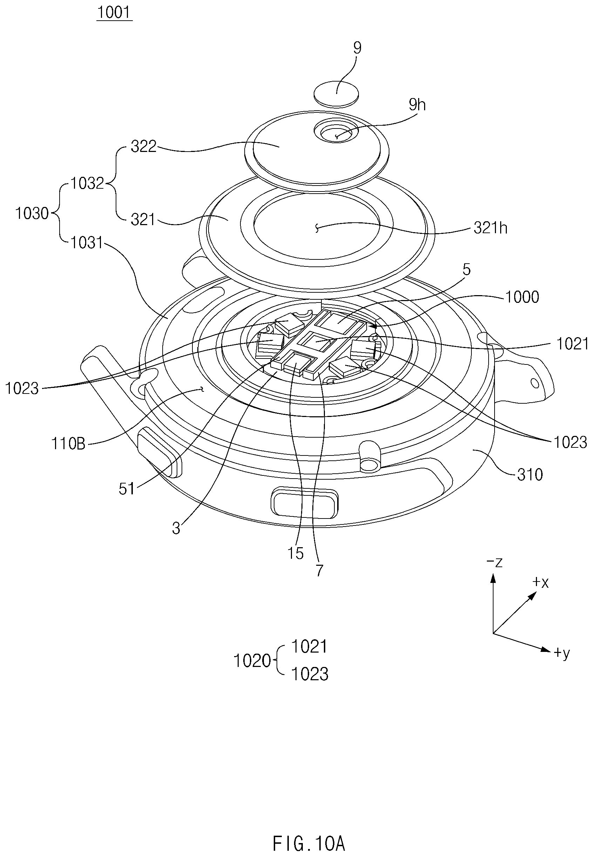

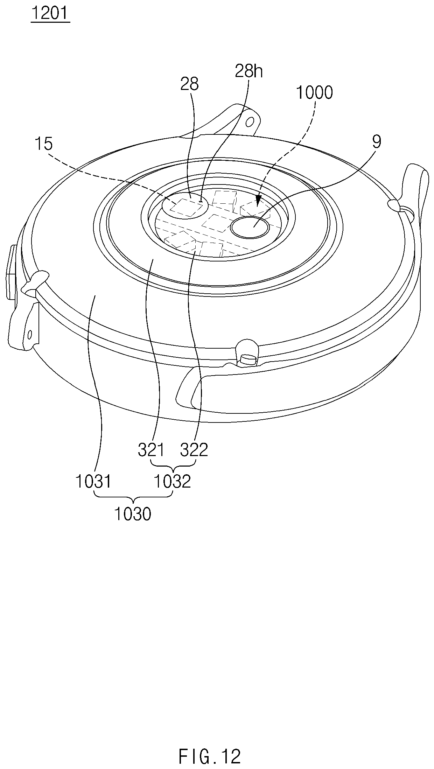

In an embodiment, the electronic device 701 may not include the second lens 728 and/or the third lens 729. For example, as illustrated in FIG. 10A, the electronic device may not include lenses corresponding to the second lens 728 and the third lens 729, and may include only a first lens 9 corresponding to the first lens 709. As another example, as illustrated in FIG. 12, the electronic device may include only the first lens 9 corresponding to the first lens 709, and a second lens 28 corresponding to the second lens 728 or the third lens 729. When the second lens 728 and/or the third lens 729 are omitted, the second sub-member 7322 may not include the second seating hole 728h and/or the third seating hole 729h, and may include only the first seating hole 709h, in which the first lens 709 is seated. When the second lens 728 and the third lens 729 are omitted, the first light emitting parts 721 of the biometric sensor module 700 may be covered by the second sub-member 7322. The light emitted from the first light emitting parts 721 may pass through the second sub-member 7322 and may reach the body of the user.

In an embodiment, a location of the first biometric sensor 705 may be different from the illustration of FIG. 7B. For example, unlike FIG. 7B, in which the first biometric sensor 705 is located between the light emitting parts 721, the light emitting part 1021 may be located between the first biometric sensor 5 and the third sensor 15 as in FIG. 10A.

The electronic device 701 according to an embodiment may include a control circuit (or a driver integrated circuit (IC) 725) for an operation of the biometric sensor module 700. The control circuit 725 may be disposed in the board 703, and may be operatively or electrically connected to the first light receiving parts 723, the first light emitting parts 721, and the first biometric sensor 705. Although FIG. 7D illustrates that the control circuit 725 is disposed in the board 703 to be located on an opposite surface to the first biometric sensor 705, the disclosure is not limited in this respect. For example, the control circuit 725 may be disposed on the same surface as the first biometric sensor 705 or may be disposed on a separate board (not illustrated) that is different from the board 703.

FIG. 8A is a view illustrating an example first lens according to various embodiments. FIG. 8B is a view illustrating an example first lens according to various embodiments.

Referring to FIG. 8A, the first lens 709 according to an embodiment may include the plurality of holes 810 (or a plurality fine holes). The plurality of holes 810 may be spaced apart from each other to be formed in a direction that is substantially perpendicular to the first lens 709. For example, the plurality of holes 810 may extend from a first surface 709A of the first lens 709 to a second surface 709B in an opposite direction to the first surface 709A. The plurality of holes 810 may pass through the first lens 709. In an embodiment, the first biometric sensor (e.g., the first biometric sensor 400 of FIG. 4D or the first biometric sensor 705 of FIG. 7B) disposed below the first lens 709 (e.g., below the second surface 709b) may be aligned with the plurality of holes 810. For example, the first biometric sensor may at least partially overlap the plurality of holes 810 of the first lens 709.

In an embodiment, the plurality of holes 810 may be fine punch holes formed through laser machining. In an embodiment, a diameter of each of the plurality of holes 810, for example, may be about 20 µm, but the disclosure is not limited in this respect.

In an embodiment, the first lens 709 may include a silicon lens or a glass lens.

In an embodiment, due to the plurality of holes 810 formed in the first lens 709, a transmissivity of a wavelength band (e.g., an LWIR band) required by the first biometric sensor 705 may be increased. In an embodiment, the first lens 709 having the plurality of holes 810 may satisfy mechanical characteristics (e.g., a hardness) required by the housing that defines the rear surface 110B of the electronic device 701 and optical characteristics (e.g., a transmissivity of an LWIR band) required by the first biometric sensor 705.

In an embodiment, an optic member may be filled in interiors of the plurality of holes 810. For example, the optic member may include a resin (e.g., a silicon resin) that satisfies a transmissivity required by the first biometric sensor 705. According to an embodiment, because the plurality of holes 810 are filled with the optic member, foreign substances (e.g., moisture, dust, skin tissues of the user, cosmetic substances, and the like) may be prevented or reduced from being introduced into the interior of the electronic device 701 through the plurality of holes 810.

In the disclosure, the plurality of holes 810 provided in the first lens 709 and the optic member filled therein may referenced as "a fourth waterproof structure" in that foreign substances, such as dust and moisture, may be prevented or reduced from being introduced through the first seating hole 709h (or the plurality of holes 810 formed in the first lens 709), in which the first lens 709 is disposed.

In another embodiment, referring to FIG. 8B, a first protection layer 820 may be disposed on the first surface 709A of the first lens 709. Because the first protection layer 820 is disposed on the first surface 709A of the first lens 709 corresponding to direction "B" that faces the body of the user, introduction of foreign substances through the plurality of holes 810 of the first lens 709 may be prevented or reduced. When the first protection layer 820 is disposed, the optic member filled in the plurality of holes 810 may be omitted, but the disclosure is not limited in this repsect. For example, the optic member may be filled in the plurality of holes 810 of the first lens 709, and the first protection layer 820 may be disposed on the first surface 709A of the first lens 709.

Optionally, a second protection layer 830 may be disposed on the second surface 709B of the first lens 709. The second protection layer 830 may prevent or reduce introduction of foreign substances, together with the first protection layer 820 and/or the optic member filled in the plurality of holes 810.

In an embodiment, the first protection layer 820 and the second protection layer 830 may be configured to satisfy the transmissivity of the LWIR band required by the first biometric sensor 705. For example, the first protection layer 820 and the second protection layer 830 may include an infrared ray transmitting film. In this case, the first protection layer 820 and the second protection layer 830 may be attached to the first lens 709. As another example, the first protection layer 820 and the second protection layer 830 may include a coating layer that is formed by applying and curing a solution that may transmit an infrared ray.

It may be understood that the first protection layer 820 and/or the second protection layer 830 disposed on the first surface 709A and/or the second surface 709B of the first lens 709 may be additionally or optionally included in "the fourth waterproof structure" in that foreign substances, such as moisture, may be prevented or reduced from being introduced through the first seating hole 709h (or the plurality of holes 810 formed in the first lens 709), in which the first lens 709 is disposed, together with the optic member filled in the plurality of holes 810 of the first lens 709.

The first protection layer 820 disposed on the plurality of holes 810 formed in the first lens 709 and the first surface 709A of the first lens 709 may be referred to, for example, as "a fifth waterproof structure" in that foreign substances, such as moisture, may be prevented or reduced from being introduced through the first seating hole 709h (the plurality of holes 810 formed in the first lens 709), in which the first lens 709 is disposed.

It may be understood that the second protection layer 830 disposed on the second surface 709B of the first lens 709 is additionally or optionally included in "the fifth waterproof structure" in that foreign substances, such as moisture, may be prevented or reduced from being introduced through the first seating hole 709h (or the plurality of holes 810 formed in the first lens 709), in which the first lens 709 is disposed, together with the first protection layer 820.

In the disclosure, the description made with reference to the first lens 709 may be applied even to the lens 409, the second lens 728, and the third lens 729, in substantially the same, similar, or corresponding manner. For example, a plurality of holes (e.g., the plurality of holes 810) may be formed in at least one of the lens 409, the second lens 728, and the third lens 729, and the optic member may be filled in the plurality of holes. As another example, the optic member may be filled in the plurality of holes of at least one of the lens 409, the second lens 728, and the third lens 729, and the first protection layer (e.g., the first protection layer 820) and/or the second protection layer (e.g., the second protection layer 830) may be disposed on the first surface (e.g., the first surface 409A of FIG. 6 and the first surface 709A of FIG. 8B) and/or the second surface (e.g., the second surface 409B of FIG. 6 and the second surface 709B of FIG. 8B), at least one of which faces the body of the user. As another example, the plurality of holes may be provided in at least one of the lens 409, the second lens 728, and the third lens 729, and the first protection layer may be disposed on the first surface. Additionally or optionally, the second protection layer may be disposed on the second surface of at least one of the lens 409, the second lens 728, and the third lens 729.

In the disclosure, although the term "waterproof" as in the first waterproof structure 10, the first waterproof member 11, and the waterproof adhesion layer 21, has been used, this term is for the purpose of denoting them or distinguishing them from other components, and the technical features are not limited by the corresponding term. For example, foreign substances that are to be blocked through the first waterproof structure 10 are not limited to only "moisture" by the term "waterproof". In this aspect, the first waterproof structure 10, the second waterproof structure 20, and the third waterproof structure 30, the fourth waterproof structure described with reference to FIG. 8A, the fifth waterproof structure, and the sixth waterproof structure described with reference to FIG. 8B may be referenced as "a foreign substance blocking structure" or "a foreign substance introduction preventing structure".

A wearable electronic device (e.g., the electronic device 301 of FIG. 3) according to an embodiment includes a housing (e.g., the housing 110 of FIG. 1) including a rear cover (e.g., the rear cover 430 of FIG. 4D or the rear cover 730 of FIG. 7D) having a first hole (e.g., the first hole H1 of FIG. 4D or the first seating hole 709h of FIG. 7D) and at least partially contacting a body of a user while the wearable electronic device is worn by the user, a first biometric sensor (e.g., the first biometric sensor 400 of FIG. 4D or the first biometric sensor 705 of FIG. 7D) disposed in the housing and aligned with the first hole, wherein the first biometric sensor includes a first lens (e.g., the lens 409 of FIG. 4D or the first lens 709 of FIG. 7D) disposed in the first hole and is configured to detect first biometric information (e.g., biometric information that represents body information) of the user based on light of a designated wavelength band (e.g., an LWIR wavelength band), which passes through the first lens, a second biometric sensor (e.g., the second biometric sensor 420 of FIG. 4C or the second biometric sensor 720 of FIG. 7D) disposed in the housing and disposed to face the rear cover, wherein the second biometric sensor is configured to detect second biometric information (e.g., biometric information that represents a heartbeat rate and/or an oxygen saturation) that is different from the first biometric information, and a blocking structure (e.g., the first waterproof structure 10 of FIG. 4D, the second waterproof structure 20 of FIG. 5D, the third waterproof structure 30 of FIG. 5C, and the fourth waterproof structure or the fifth waterproof structure described with reference to FIGS. 8A and 8B) that prevents or reduces (blocks) foreign substances from being introduced through the first hole.

In an embodiment, the first biometric sensor may be configured to detect a temperature as the first biometric information, based on the light of the designated wavelength band, which is input into the first hole.

In an embodiment, the rear cover may include a first member (e.g., the first member 431 of FIG. 4D or the first member 731 of FIG. 7D) and a second member (e.g., the second member 432 of FIG. 4D of the second member 732 of FIG., 7D) disposed under the first member, the second member may be coupled to the first member to close an opening formed at a central portion of the first member, the first hole may be formed in the first member to be exposed through a rear surface (e.g., the rear surface 110B of FIG. 4D or the rear surface 110B of FIG. 7D) of the wearable electronic device, and the second biometric sensor may be at least partially disposed in the opening to face the second member.

In an embodiment, the first biometric sensor may include a board (e.g., the board 403 of FIG. 4A), a sensor part (e.g., the sensor part 405 of FIG. 4A) disposed in the board, wherein the sensor part is disposed to face the first lens to receive the light of the designated wavelength band, which passes through the first lens, and a sensor housing (e.g., the sensor housing 407 of FIG. 4A) surrounding the sensor part, and having a light receiving hole (e.g., the light receiving hole OP3 of FIG. 4), to which the first lens is coupled.

In an embodiment, the wearable electronic device includes a printed circuit board (e.g., the printed circuit board 380 of FIG. 4A), in which at least one processor (e.g., the processor 920 of FIG. 9) is operatively connected to the first biometric sensor and the second biometric sensor, the first biometric sensor may include an elastic member (e.g., the elastic member 402 of FIG. 4A) disposed between the board and the printed circuit board, and the elastic member may provide an elastic force in a direction that faces the lens, to the board.

In an embodiment, the blocking structure may include a first blocking structure (e.g., the first waterproof structure 10 of FIG. 4D), the sensor housing may include a first part (e.g., the first part 4071 of FIG. 4D) disposed on the board to surround the sensor part, a second part (e.g., the second part 4072 of FIG. 4D) extending from an inner periphery of the first part along the sensor part, and a third part (e.g., the third part 4073 of FIG. 4D) extending from the second part toward the first lens, and defining the light receiving hole, and the first blocking structure may include a first waterproof member (e.g., the first waterproof member 11 of FIG. 4D) disposed between the first part and the first member, and an adhesion member (e.g., the adhesion member 12 of FIG. 4D) disposed between the first waterproof member and the first member.

In an embodiment, the first waterproof member may include a boss (e.g., the boss 13 of FIG. 14) protruding toward the first part and compressed by the first part.

In an embodiment, the first waterproof member may include rubber.

In an embodiment, the blocking structure may include a second blocking structure (e.g., the second waterproof structure 20 of FIG. 5B), and the second blocking structure may include a waterproof adhesion layer (e.g., the waterproof adhesion layer 21 of FIG. 5B) interposed between the sensor housing and the first member.

In an embodiment, the sensor housing may include a first part (e.g., the first part 4071 of FIG. 5B) disposed on the board to surround the sensor part, a second part (e.g., the second part 4072 of FIG. 5B) extending from an inner periphery of the first part along the sensor part, and a third part (e.g., the third part 4073 of FIG. 5B) extending from the second part toward the first lens, and defining the light receiving hole, and the second part and the third part may be at least partially inserted into the first hole, and the waterproof adhesion layer may include a first layer (e.g., the first layer 211 of FIG. 5B) interposed between the first part and the first member, and a second layer (e.g., the second layer 212 of FIG. 5B) interposed between a first inner peripheral surface (e.g., the first inner peripheral surface S1 of FIG. 5B) of the first hole and the second part, and between the first inner peripheral surface and the third part.

In an embodiment, the blocking structure may include a third blocking structure (e.g., the third waterproof structure 30 of FIG. 5C), and the sensor housing may include a first part (e.g., the first part 4071 of FIG. 5C) disposed on the board to surround the sensor part, a second part (e.g., the second part 4072 of FIG. 5C) extending from an inner periphery of the first part along the sensor part, a third part (e.g., the third part 4073 of FIG. 5C) extending from the second part toward the first lens, and defining the light receiving hole, and a protrusion (e.g., the protrusion 4072a of FIG. 5C) extending from the second part to face the first part, and the third blocking structure may include a second waterproof member (e.g., the second waterproof member 31 of FIG. 5C) interposed between a recess (e.g., the recess 32 of FIG. 5C) defined by the first part, the second part, and the protrusion, and the first member.

In an embodiment, the second waterproof member may include an O-ring.

In an embodiment, the first lens may include a first surface (e.g., the first surface 409A of reference numeral 601 of FIG. 6) that faces an outside (e.g., direction "B") of the wearable electronic device, and the first lens may be disposed in an interior of the first hole such that the first surface is spaced apart from a rear surface (e.g., the rear surface 110B of reference numeral 601 of FIG. 6) of the wearable electronic device.

In an embodiment, the first lens may include a first surface (e.g., the first surface 409A of reference numeral 603 of FIG. 6) that faces an outside (e.g., direction "B") of the wearable electronic device, and the first surface of the first lens may extend substantially with no step from the rear cover in the first hole to define a rear surface (e.g., the rear surface 110B of reference numeral 603 of FIG. 6) of the wearable electronic device together with the rear cover.

In an embodiment, the blocking structure may include a fourth blocking structure (e.g., the fourth waterproof structure of FIGS. 8A and 8B), and the fourth blocking structure may include a plurality of fine holes (e.g., the plurality of holes 810 of FIG. 8A) formed in the first lens, and optic members filled in the plurality of fine holes, respectively.

In an embodiment, the optic members may include a resin that transmits the light of the designated wavelength band.

In an embodiment, the first lens may include a first surface (e.g., the first surface 709a of FIG. 8B) that faces an outside of the wearable electronic device, and a second surface (e.g., the second surface 709b of FIG. 8B) in an opposite direction to the first surface, the first lens may have a plurality of fine holes (e.g., the plurality of holes 810 of FIG. 8A) that extend from the first surface to the second surface and pass through the first lens, and the wearable electronic device may include a first protection layer (e.g., the first protection layer 820 of FIG. 8B) and/or a second protection layer (e.g., the second protection layer 830 of FIG. 8B) disposed on the first surface and/or the second surface of the first lens.

In an embodiment, the first protection layer and/or the second protection layer may include a film or coating layer configured to transmit the light of the designated wavelength band.

In an embodiment, the wearable electronic device may include optic members filled in the plurality of fine holes, respectively.

A wearable electronic device (e.g., the electronic device 701 of FIG. 7A) according to an embodiment of the disclosure includes a frame (e.g., the frame 310 of FIG. 7A), a display (e.g., the display 320 of FIG. 7A) disposed on one side of the frame, a rear cover (e.g., the rear cover 730 of FIG. 7A) disposed on an opposite side of the frame, and having a first seating hole (e.g., the first seating hole 709h of FG. 7A), a second seating hole (e.g., the second seating hole 728h of FIG. 7A), and a third seating hole (e.g., the third seating hole 729h of FIG. 7A), a printed circuit board (e.g., the printed circuit board 380 of FIG. 7A) disposed between the display and the rear cover, a processor (e.g., the processor 920 of FIG. 9) disposed in the printed circuit board, and a biometric sensor module (e.g., the biometric sensor module 700 of FIG. 7B) disposed between the printed circuit board and the rear cover, and including a first biometric sensor (e.g., the first biometric sensor 705 of FIG. 7B) and a second biometric sensor (e.g., the second biometric sensor 720 of FIG. 7B) operatively connected to the processor, wherein the second biometric sensor includes a first light emitting part (e.g., among the first light emitting parts 721 of FIG. 7B, the light emitting part disposed in the second recess R2), a second light emitting part (e.g., among the first light emitting parts 721 of FIG. 7B, the light emitting part disposed in the third recess R3), and a plurality of light receiving parts (e.g., the first light receiving parts 723 of FIG. 7B) that overlap the rear cover, a first lens (e.g., the first lens 709 of FIG. 7A) disposed in the first seating hole, and at least partially overlapping the first biometric sensor, a second lens (e.g., the second lens 728 of FIG. 7A) disposed in the second seating hole, and at least partially overlapping the first light emitting part of the first biometric sensor, and a third lens (e.g., the third lens 729 of FIG. 7A) disposed in the third seating hole, and at least partially overlapping the second light emitting part of the second biometric sensor, and the processor is configured to detect a body temperature of a user as first biometric information, based on light of a designated wavelength band (e.g., an LWIR wavelength band), which is received using the first biometric sensor, and second biometric information that is different from the first biometric information, using the second biometric sensor.

In an embodiment, the first lens may include a plurality of fine holes (e.g., the plurality of holes 810 of FIG. 8A), and optic members filled in the plurality of fine holes, respectively.

In an embodiment, the first lens may be located between the second lens and the third lens, the first lens, the second lens, and the third lens may be at least partially aligned along one direction (e.g., a direction corresponding to line B-B' of FIG. 7B), and the wearable electronic device may include a first protection layer (e.g., the first protection layer 820 of FIG. 8B) and/or a second protection layer (e.g., the second protection layer 830 of FIG. 8B) and/or a second protection layer disposed on a first surface (e.g., the first surface 709a of FIG. 8B) of the first lens, which is exposed through the first seating hole, and/or a second surface (e.g., the second surface 709b of FIG. 8B) that is opposite to the first surface.

FIG. 9 is a block diagram illustrating an electronic device 901 in a network environment 900 according to various embodiments. Referring to FIG. 9, the electronic device 901 in the network environment 900 may communicate with an electronic device 902 via a first network 998 (e.g., a short-range wireless communication network), or at least one of an electronic device 904 or a server 908 via a second network 999 (e.g., a long-range wireless communication network). According to an embodiment, the electronic device 901 may communicate with the electronic device 904 via the server 908. According to an embodiment, the electronic device 901 may include a processor 920, memory 930, an input module 950, a sound output module 955, a display module 960, an audio module 970, a sensor module 976, an interface 977, a connecting terminal 978, a haptic module 979, a camera module 980, a power management module 988, a battery 989, a communication module 990, a subscriber identification module (SIM) 996, or an antenna module 997. In various embodiments, at least one of the components (e.g., the connecting terminal 978) may be omitted from the electronic device 901, or one or more other components may be added in the electronic device 901. In various embodiments, some of the components (e.g., the sensor module 976, the camera module 980, or the antenna module 997) may be implemented as a single component (e.g., the display module 960).

The processor 920 may execute, for example, software (e.g., a program 940) to control at least one other component (e.g., a hardware or software component) of the electronic device 901 coupled with the processor 920, and may perform various data processing or computation. According to an embodiment, as at least part of the data processing or computation, the processor 920 may store a command or data received from another component (e.g., the sensor module 976 or the communication module 990) in volatile memory 932, process the command or the data stored in the volatile memory 932, and store resulting data in non-volatile memory 934. According to an embodiment, the processor 920 may include a main processor 921 (e.g., a central processing unit (CPU) or an application processor (AP)), or an auxiliary processor 923 (e.g., a graphics processing unit (GPU), a neural processing unit (NPU), an image signal processor (ISP), a sensor hub processor, or a communication processor (CP)) that is operable independently from, or in conjunction with, the main processor 921. For example, when the electronic device 901 includes the main processor 921 and the auxiliary processor 923, the auxiliary processor 923 may be adapted to consume less power than the main processor 921, or to be specific to a specified function. The auxiliary processor 923 may be implemented as separate from, or as part of the main processor 921.

The auxiliary processor 923 may control at least some of functions or states related to at least one component (e.g., the display module 960, the sensor module 976, or the communication module 990) among the components of the electronic device 901, instead of the main processor 921 while the main processor 921 is in an inactive (e.g., sleep) state, or together with the main processor 921 while the main processor 921 is in an active state (e.g., executing an application). According to an embodiment, the auxiliary processor 923 (e.g., an image signal processor or a communication processor) may be implemented as part of another component (e.g., the camera module 980 or the communication module 990) functionally related to the auxiliary processor 923. According to an embodiment, the auxiliary processor 923 (e.g., the neural processing unit) may include a hardware structure specified for artificial intelligence model processing. An artificial intelligence model may be generated by machine learning. Such learning may be performed, e.g., by the electronic device 901 where the artificial intelligence is performed or via a separate server (e.g., the server 908). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted Boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 930 may store various data used by at least one component (e.g., the processor 920 or the sensor module 976) of the electronic device 901. The various data may include, for example, software (e.g., the program 940) and input data or output data for a command related thereto. The memory 930 may include the volatile memory 932 or the non-volatile memory 934.

The program 940 may be stored in the memory 930 as software, and may include, for example, an operating system (OS) 942, middleware 944, or an application 946.

The input module 950 may receive a command or data to be used by another component (e.g., the processor 920) of the electronic device 901, from the outside (e.g., a user) of the electronic device 901. The input module 950 may include, for example, a microphone, a mouse, a keyboard, a key (e.g., a button), or a digital pen (e.g., a stylus pen).

The sound output module 955 may output sound signals to the outside of the electronic device 901. The sound output module 955 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 960 may visually provide information to the outside (e.g., a user) of the electronic device 901. The display module 960 may include, for example, a display, a hologram device, or a projector and control circuitry to control a corresponding one of the display, hologram device, and projector. According to an embodiment, the display module 960 may include a touch sensor adapted to detect a touch, or a pressure sensor adapted to measure the intensity of force incurred by the touch.

The audio module 970 may convert a sound into an electrical signal and vice versa. According to an embodiment, the audio module 970 may obtain the sound via the input module 950, or output the sound via the sound output module 955 or a headphone of an external electronic device (e.g., an electronic device 902) directly (e.g., wiredly) or wirelessly coupled with the electronic device 901.

The sensor module 976 may detect an operational state (e.g., power or temperature) of the electronic device 901 or an environmental state (e.g., a state of a user) external to the electronic device 901, and then generate an electrical signal or data value corresponding to the detected state. According to an embodiment, the sensor module 976 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 977 may support one or more specified protocols to be used for the electronic device 901 to be coupled with the external electronic device (e.g., the electronic device 902) directly (e.g., wiredly) or wirelessly. According to an embodiment, the interface 977 may include, for example, a high definition multimedia interface (HDMI), a universal serial bus (USB) interface, a secure digital (SD) card interface, or an audio interface.

A connecting terminal 978 may include a connector via which the electronic device 901 may be physically connected with the external electronic device (e.g., the electronic device 902). According to an embodiment, the connecting terminal 978 may include, for example, a HDMI connector, a USB connector, a SD card connector, or an audio connector (e.g., a headphone connector).

The haptic module 979 may convert an electrical signal into a mechanical stimulus (e.g., a vibration or a movement) or electrical stimulus which may be recognized by a user via his tactile sensation or kinesthetic sensation. According to an embodiment, the haptic module 979 may include, for example, a motor, a piezoelectric element, or an electric stimulator.

The camera module 980 may capture a still image or moving images. According to an embodiment, the camera module 980 may include one or more lenses, image sensors, image signal processors, or flashes.

The power management module 988 may manage power supplied to the electronic device 901. According to an embodiment, the power management module 988 may be implemented as at least part of, for example, a power management integrated circuit (PMIC).

The battery 989 may supply power to at least one component of the electronic device 901. According to an embodiment, the battery 989 may include, for example, a primary cell which is not rechargeable, a secondary cell which is rechargeable, or a fuel cell.

The communication module 990 may support establishing a direct (e.g., wired) communication channel or a wireless communication channel between the electronic device 901 and the external electronic device (e.g., the electronic device 902, the electronic device 904, or the server 908) and performing communication via the established communication channel. The communication module 990 may include one or more communication processors that are operable independently from the processor 920 (e.g., the application processor (AP)) and supports a direct (e.g., wired) communication or a wireless communication. According to an embodiment, the communication module 990 may include a wireless communication module 992 (e.g., a cellular communication module, a short-range wireless communication module, or a global navigation satellite system (GNSS) communication module) or a wired communication module 994 (e.g., a local area network (LAN) communication module or a power line communication (PLC) module). A corresponding one of these communication modules may communicate with the external electronic device via the first network 998 (e.g., a short-range communication network, such as Bluetooth^{™}, wireless-fidelity (Wi-Fi) direct, or infrared data association (IrDA)) or the second network 999 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 992 may identify and authenticate the electronic device 901 in a communication network, such as the first network 998 or the second network 999, using subscriber information (e.g., international mobile subscriber identity (IMSI)) stored in the subscriber identification module 996.

The wireless communication module 992 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 992 may support a high-frequency band (e.g., the mmWave band) to achieve, e.g., a high data transmission rate. The wireless communication module 992 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 992 may support various requirements specified in the electronic device 901, an external electronic device (e.g., the electronic device 904), or a network system (e.g., the second network 999). According to an embodiment, the wireless communication module 992 may support a peak data rate (e.g., 20Gbps or more) for implementing eMBB, loss coverage (e.g., 964dB or less) for implementing mMTC, or U-plane latency (e.g., 0.5ms or less for each of downlink (DL) and uplink (UL), or a round trip of 9ms or less) for implementing URLLC.

The antenna module 997 may transmit or receive a signal or power to or from the outside (e.g., the external electronic device) of the electronic device 901. According to an embodiment, the antenna module 997 may include an antenna including a radiating element composed of or including a conductive material or a conductive pattern formed in or on a substrate (e.g., a printed circuit board (PCB)). According to an embodiment, the antenna module 997 may include a plurality of antennas (e.g., array antennas). In such a case, at least one antenna appropriate for a communication scheme used in the communication network, such as the first network 998 or the second network 999, may be selected, for example, by the communication module 990 (e.g., the wireless communication module 992) from the plurality of antennas. The signal or the power may then be transmitted or received between the communication module 990 and the external electronic device via the selected at least one antenna. According to an embodiment, another component (e.g., a radio frequency integrated circuit (RFIC)) other than the radiating element may be additionally formed as part of the antenna module 997.

According to various embodiments, the antenna module 997 may form a mmWave antenna module. According to an embodiment, the mmWave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 901 and the external electronic device 904 via the server 908 coupled with the second network 999. Each of the electronic devices 902 or 904 may be a device of a same type as, or a different type, from the electronic device 901. According to an embodiment, all or some of operations to be executed at the electronic device 901 may be executed at one or more of the external electronic devices 902, 904, or 908. For example, if the electronic device 901 should perform a function or a service automatically, or in response to a request from a user or another device, the electronic device 901, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 901. The electronic device 901 may provide the outcome, with or without further processing of the outcome, as at least part of a reply to the request. To that end, a cloud computing, distributed computing, mobile edge computing (MEC), or client-server computing technology may be used, for example. The electronic device 901 may provide ultra low-latency services using, e.g., distributed computing or mobile edge computing. In another embodiment, the external electronic device 904 may include an internet-ofthings (IoT) device. The server 908 may be an intelligent server using machine learning and/or a neural network. According to an embodiment, the external electronic device 904 or the server 908 may be included in the second network 999. The electronic device 901 may be applied to intelligent services (e.g., smart home, smart city, smart car, or healthcare) based on 5G communication technology or IoT-related technology.

The electronic device according to various embodiments may be one of various types of electronic devices. The electronic devices may include, for example, a portable communication device (e.g., a smartphone), a computer device, a portable multimedia device, a portable medical device, a camera, a wearable device, a home appliance, or the like. According to an embodiment of the disclosure, the electronic devices are not limited to those described above.

It should be appreciated that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and " at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), the element may be coupled with the other element directly (e.g., wiredly), wirelessly, or via a third element.

As used in connection with various embodiments of the disclosure, the term "module" may include a unit implemented in hardware, software, or firmware, or any combination thereof, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 940) including one or more instructions that are stored in a storage medium (e.g., internal memory 936 or external memory 938) that is readable by a machine (e.g., the electronic device 901). For example, a processor (e.g., the processor 920) of the machine (e.g., the electronic device 901) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a compiler or a code executable by an interpreter. The machine-readable storage medium may be provided in the form of a non-transitory storage medium. The term "non-transitory" storage medium may refer, for example, to a tangible device, and may not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

The description of the electronic device 901 made with reference to FIG. 9 also may be applied to an electronic device 1001 and an electronic device 1201, which will be described below in the same way.

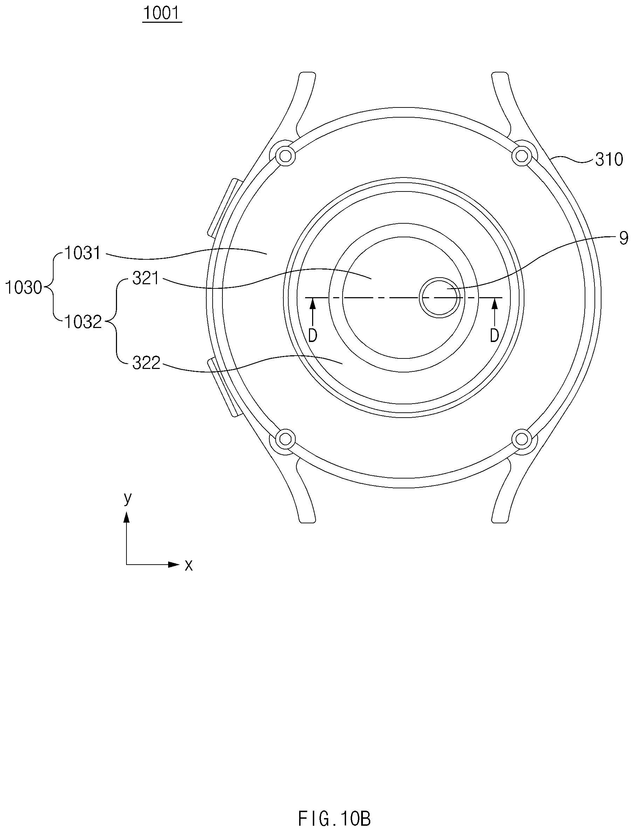

FIG. 10A is a view illustrating an example electronic device according to various embodiments. FIG. 10B is a view illustrating an example electronic device according to various embodiments.

Referring to FIGS. 10A and 10B, the electronic device 1001 (e.g., the electronic device 701 of FIG. 7A) according to an embodiment may include a rear cover 1030 (e.g., the rear cover 730 of FIG. 7A). The rear cover 1030 may include a first member 1031 (e.g., the first member 731 of FIG. 7A) and a second member 1032 (e.g., the second member 732 of FIG. 7A). The first member 1031 may be coupled to the frame 310. The second member 1032 may be coupled to the first member 1031. The second member 1032 may at least partially close a hollow formed in the first member 1031.

In an embodiment, the second member 1032 may include a first sub-member 321 (e.g., the first sub-member 7321 of FIG. 7A) and a second sub-member 322 (e.g., the second sub-member 7322 of FIG. 7A). The first sub-member 321 may be coupled to the first member 1031. The second sub-member 322 may be coupled to the first sub-member 321. A hollow 321h may be formed in the first sub-member 321. The second sub-member 322 may at least partially close the hollow 321h of the first sub-member 321. A first seating hole 9h (e.g., the first seating hole 709h of FIG. 7A) may be formed in the second sub-member 322. The first seating hole 9h may pass through the second sub-member 322. The first sub-member 321 may be formed of or include glass, a resin, ceramics, a metal, or a combination thereof. The second sub-member 322 may include glass. For example, the second sub-member 322 may include glass formed of or including a silica-based material. As another example, the second sub-member 322 may be formed of or include an alumina-based material (for example, sapphire glass).

In an embodiment, the electronic device 1001 may include a biometric sensor module 1000 (e.g., the biometric sensor module 700 of FIG. 7A). The biometric sensor module 1000 may be disposed in the frame 310. The biometric sensor module 1000 may be at least partially accommodated in the hollow formed in the first member 1031. The biometric sensor module 1000 may be located below (e.g., in the +z direction) of the rear cover 1030. The biometric sensor module 1000 may include a plurality of sensors. The biometric sensor module 1000 may be disposed such that the plurality of sensors face the rear cover 1030. For example, the biometric sensor module 1000 may be disposed such that the plurality of sensors face the second sub-member 322 of the rear cover 1030. The biometric sensor module 1000 may at least partially overlap the second sub-member 322. The biometric sensor module 1000 may detect the first biometric information (e.g., a body temperature) and the second biometric information (e.g., a heart rate and/or an oxygen saturation in blood).

In an embodiment, the biometric sensor module 1000 may include a substrate 3, a partition wall structure 7, a first biometric sensor 5, a second biometric sensor 1020, a third sensor 15, and a light shielding member 51.

The partition wall structure 7, the first biometric sensor 5, the second biometric sensor 1020, and the third sensor 15 may be disposed on one surface (e.g., the one surface 703A of FIG. 7B) of the substrate 3 (e.g., the substrate 703 of FIG. 7B). The first biometric sensor 5 may correspond to the first biometric sensor 705 of FIG. 7B. For example, the first biometric sensor 5 may detect the first biometric information (e.g., the body temperature) of the user, based on an infrared ray (or infrared ray energy) emitted from the body of the user. The second biometric sensor 1020 may correspond to the second biometric sensor 720 of FIG. 7B. For example, the second biometric sensor 1020 may include at least one light emitting part 1021 (e.g., the first light emitting parts 721 of FIG. 7B), and at least one light receiving part 1023 (e.g., the first light receiving parts 723 of FIG. 7B). The second biometric sensor 1020 may detect the second biometric information (e.g., a heartbeat rate and/or an oxygen saturation) using the first light emitting part 1021 and the first light receiving part 1023.

The third sensor 15, for example, may include a light emitting part (e.g., the first light emitting part 721 of FIG. 7B) for the second biometric sensor 1020. As another example, the third sensor 15 may include at least one sensor that is different from the first biometric sensor 5 and/or the second biometric sensor 1020. The at least one sensor of the third sensor 15 may be configured to detect third biometric information that is different from the first biometric information and/or the second biometric information.

The third biometric information, for example, may include temperature information (e.g., a body temperature). The third biometric information, as another example, may include blood glucose information. The blood glucose information may be acquired by detecting a change in a substance (e.g., glucose) in blood that indicates a blood glucose value of a user, using light, but the disclosure is not limited in this respect. The third biometric information may include information that indicates whether an external object is close and/or whether the close external object is a living organism. The third sensor 15, for example, may include an ultrasonic wave sensor to acquire the third biometric information, but the disclosure is not limited in this respect.

A partition wall structure 7 (e.g., the partition wall structure 707 of FIG. 7B) may extend from the substrate 3 toward the rear cover 1030. The partition wall structure 7 may extend from the substrate 3 in a height direction (e.g., the -z direction) of the electronic device 1001. The partition wall structure 7 may define recesses (e.g., the first, second, and third recesses R1, R2, and R3 of FIG. 7B) that accommodate the first biometric sensor 5, the light emitting part 1021, and the third sensor 15, respectively. The partition wall structure 7 may at least partially surround the first biometric sensor 5, the light emitting part 1021, and the third sensor 15.

In an embodiment, the first biometric sensor 5, the light emitting part 1021 of the second biometric sensor 1020, and the third sensor 15 may be at least partially aligned with respect to one direction (e.g., the x axis direction). The light emitting part 1021 of the second biometric sensor 1020 may be disposed between the first biometric sensor 5 and the third sensor 15. The light emitting part 1021 and the light receiving part 1023 may be disposed with the partition wall structure 7 being interposed therebetween. For example, the light emitting part 1021 may be disposed on an inner side of the partition wall structure 7, and the light receiving part 1023 may be disposed on an outer side of the partition wall structure 7.

In an embodiment, the light shielding member 51 (e.g., the light shielding member 51 of FIG. 7B) may be disposed on the partition wall structure 7. The light shielding member 51 may be interposed between the partition wall structure 7 and the rear cover 1030, and may seal a gap between the rear cover 1030 and the partition wall structure 7.

The electronic device 1001 according to an embodiment may include the first lens 9. The description of the lens 409 and the first lens 709 may be applied to the first lens 9 in substantially a same, similar, or corresponding scheme. For example, the first lens 9 may be disposed in the first seating hole 9h formed in the second sub-member 322. The first lens 9 may include a glass lens or a silicon lens.

The first seating hole 9h of the second sub-member 322 may be formed at a location corresponding to the first biometric sensor 5. For example, when the rear cover 1030 is viewed from a front side (e.g., when viewed in the z axis direction), the first seating hole 9h may at least partially be aligned with or overlap the first biometric sensor 5. The first lens 9 disposed in the first seating hole 9h may correspond to the first biometric sensor 5. For example, when the rear cover 1030 is viewed from a front side (e.g., when viewed in the z axis direction), the first lens 9 may at least partially be aligned with the first biometric sensor 5. The first biometric sensor 5 may receive light delivered through the first lens 9. The first biometric sensor 5 may detect the first biometric information (e.g., a temperature) based on the light received through the first lens 9.

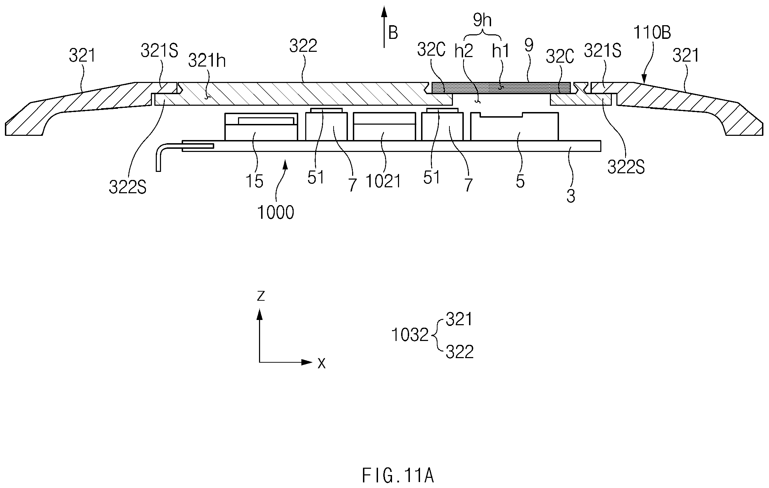

FIG. 11A is a cross-sectional view illustrating an example electronic device according to various embodiments. FIG. 11A is a cross-sectional view corresponding to line D-D' of FIG. 10B. In FIG. 11A, illustration of configurations, except for the first lens 9, the second member 1032, the biometric module 1000, is omitted for convenience for description.

Referring to FIG. 11A, the first seating hole 9h may include a first hole h1 and a second hole h2 that pass through the second sub-member 322. The first hole h1 and the second hole h2 may communicate with each other. The first hole h1 may be formed on a rear surface 110B of the second sub-member 322. The second hole h2 may be located between the first hole h1 and the first biometric sensor 5.

In an embodiment, the first hole h1 may have a diameter that is larger than that of the second hole h2. Accordingly, a seating surface 32C may be defined in the first seating hole 9h. The first lens 9 may be disposed on the seating surface 32C. The first lens 9 disposed on the seating surface 32C may be at least partially accommodated in the first hole h1. The first lens 9 may cover the second hole h2.

In an embodiment, a first waterproof adhesion member (not illustrated) may be interposed between the first lens 9 and the second sub-member 322. For example, the first waterproof adhesion member may be at least partially disposed between the first lens 9 and an inner peripheral surface of the first hole h1, and/or between the first lens 9 and the seating surface 32C. The first waterproof adhesion member, for example, may include a waterproof adhesion tape, but the disclosure is not limited in this respect. As another example, the first waterproof adhesion member may be formed by applying and curing a substantially transparent epoxy-based bonding liquid, but the disclosure is not limited in this respect. The first waterproof adhesion member may prevent or reduce foreign substances from being introduced into an interior of the electronic device 1001 through the first seating hole 9h.

In an embodiment, the first lens 9 may be coupled to the second sub-member 322 through the first waterproof adhesion member. Alternatively, the first lens 9 may be coupled to the second sub-member 322 in a scheme of being press-fitted with the first hole h1. For example, unlike the illustration, the first lens 9 may be formed such that a diameter thereof is substantially the same as that of the first hole h1. The first lens 9 may be inserted into the first hole h1 through interference-fitting, and may be coupled to the second sub-member 322. When the first lens 9 is press-fitted with the first hole h1, the first waterproof adhesion member may be omitted, but the disclosure is not limited in this respect. Even when the first waterproof adhesion member is omitted, introduction of foreign substances may be prevented or reduced through the first lens 9 press-fitted with the first seating hole 9h.

In an embodiment, a hollow 321h may be formed in the first sub-member 321. The second sub-member 322 may be disposed in the hollow 321h. A peripheral portion of the second sub-member 322 may be combined with an inner peripheral surface of the hollow 321h. The first sub-member 321 may include a first stepped part 321S formed on an inner peripheral surface of the hollow 321h, and the second sub-member 322 may include a second stepped part 322S formed at a peripheral portion thereof. The first stepped part 321S and the second stepped part 322S may be combined with each other.

In an embodiment, a second waterproof adhesion member (not illustrated) may be interposed between the first stepped part 321S and the second stepped part 322S. The second waterproof adhesion member may be at least partially formed along a surface, on which the first stepped part 321S and the second stepped part 322S face each other. The second waterproof adhesion member, for example, may include a waterproof adhesion tape, but the disclosure is not limited in this respect. As another example, the second waterproof adhesion member may be formed by applying and curing a substantially transparent epoxy-based bonding liquid, but the disclosure is not limited in this respect. The second waterproof adhesion member may prevent or reduce foreign substances from being introduced into an interior of the electronic device 1001 through the hollow 321h.

The second sub-member 322 may be coupled to the first sub-member 321 through the second waterproof adhesion member. Alternatively, the second sub-member 322 may be press-fitted in the hollow 321h through interference-fitting to be coupled to the first sub-member 1031. When second sub-member 322 is press-fitted in the hollow 321h of the first sub-member 321, the second waterproof adhesion member may be omitted, but the disclosure is not limited in this respect. Even when the second waterproof adhesion member is omitted, introduction of foreign substances may be prevented or reduced through the first sub-member 321 press-fitted with the hollow 321h.

The above-described scheme of press-fitting and coupling the first lens 9 in the first seating hole 9h of the second sub-member 322 may be applied to the first lens 709 of FIG. 7A in the same way. For example, the first lens 709 may be press-fitted in and coupled to the first seating hole 709h provided in the second sub-member 7322.

The above-described scheme of press-fitting and coupling the second sub-member 322 in the hollow 321h of the first sub-member 321 may be applied to the second sub-member 7322 of FIG. 7A in the same way. For example, the second sub-member 7322 may be press-fitted in and coupled to the hollow provided in the first sub-member 7321.

Although FIG. 11A illustrates that the light shielding member 51 and the second sub-member 322 are spaced apart from each other, the disclosure is not limited thereto. For example, the light shielding member 51 may contact the second sub-member 322.

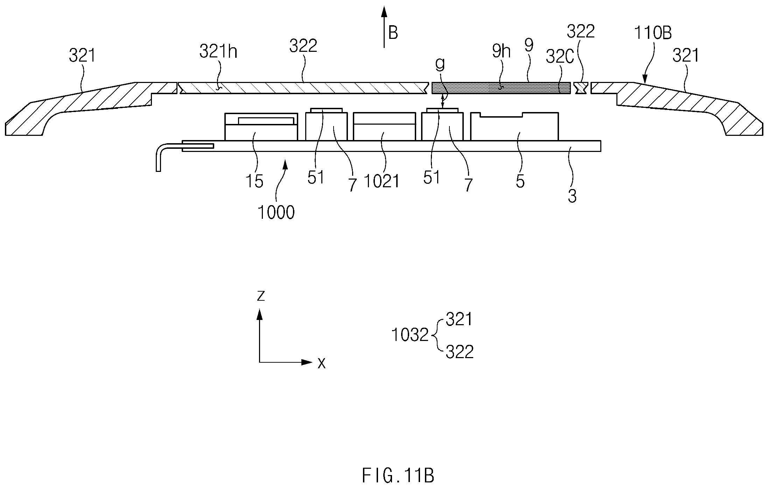

FIG. 11B is a cross-sectional view illustrating an example electronic device according to various embodiments. FIG. 11B is a cross-sectional view corresponding to line D-D' of FIG. 10B. In FIG. 11B, illustration of configurations, except for the first lens 9, the second member 1032, the biometric module 1000, is omitted for convenience for description.

Referring to FIG. 11B, the first seating hole 9h of the second sub-member 322 may be formed to have one diameter unlike the first hole h1 and the second hole h2 of FIG. 11A. The first lens 9 may be coupled to the second sub-member 322 in a scheme of being press-fitted with the first seating hole 9h. Alternatively or additionally, the first lens 9 may be coupled to the second sub-member 322 through the waterproof adhesion member interposed between the first lens 9 and an inner surface of the first seating hole 9h. The waterproof adhesion member, for example, may be formed by applying a bonding liquid including a substantially epoxy-based resin and then curing the bonding liquid, but the disclosure is not limited in this respect. In an embodiment, a gap "g" between the first lens 9 (and/or the second sub-member 322) and the partition wall structure 7 may not be provided. The partition wall structure 7 that contacts the first lens 9 and/or the second sub-member 322 may provide an area, in which the bonding liquid may be applied. For example, the partition wall structure 7 that contacts the first lens 9 and/or the second sub-member 322 may provide the same function as that of the seating surface 32C of FIG. 11A. The first lens 9 press-fitted in the second sub-member 322 may prevent or reduce introduction of foreign substances through the first seating hole 9h. Alternatively or additionally, the waterproof adhesion member interposed between the first lens 9 and the second sub-member 322 may prevent or reduce introduction of foreign substances through the first seating hole 9h.

In an embodiment, the second sub-member 322 may be press-fitted in the first sub-member 321. The second sub-member 322 may not include a stepped part (e.g., the second stepped part 322S of FIG. 11A) at a peripheral portion thereof. The first lens 321 press-fitted in the second sub-member 322 may prevent or reduce introduction of foreign substances through the hollow 321h.

FIG. 12 illustrates an example electronic device according to various embodiments. Referring to FIG. 12, an electronic device 1201 according to an embodiment may include a second lens 28 (e.g., the second lens 728 or the third lens 729 of FIG. 7A). The second lens 28 may be coupled to the second sub-member 322. The second lens 28 may be disposed in the second seating hole 28h provided in the second sub-member 322. The description of the scheme of coupling the first lens 9 and the first seating hole 9h, which has been made with reference to FIGS. 11A and 11B, may be applied to a scheme of coupling the second sub-member 322 and the second seating hole 28h in substantially the same way. For example, the second lens 28 may be press-fitted in the second seating hole 28h, and/or may be coupled to the second sub-member 322 through the waterproof adhesion member interposed therebetween.

The second lens 28 may correspond to the third sensor 15 of the biometric sensor module 1000. The second lens 28 may at least partially overlap the third sensor 15. The third sensor 15 may be disposed to face the second lens 28. The third sensor 15 may acquire the third biometric information (e.g., blood glucose information) through the second lens 28.

The wearable electronic device 701, 1001, and 1201 according to an embodiment may include the frame 310, the display 320, the rear cover 730 and 1030, the printed circuit board 380, the processor 920, the biometric sensor module 700 and 1000, and the first lens 709 and 9. The display 320 may be disposed on one side of the frame 310. The rear cover 730 and 1030 may be disposed on an opposite side of the frame 310, and the first seating hole 709h and 9h may be formed in the rear cover 730 and 1030. The printed circuit board 380 may be disposed between the display 320 and the rear cover 1030. The processor 920 may be disposed in the printed circuit board 380. The biometric sensor module 700 and 1000 may include the first biometric sensor 705 and 5 and the second biometric sensor 720 and 1020. The first biometric sensor 705 and 5 and the second biometric sensor 720 and 1020 may be disposed between the printed circuit board 380 and the rear cover 1030, and may be operatively connected to the processor 920. The second biometric sensor 720 and 1020 may include the light emitting part 721 and 1021 and the light receiving part 723 and 1023. The first lens 709 and 9 may be disposed in the first seating hole 709h and 9h and may at least partially overlap the first biometric sensor 705 and 5. The processor 920 may be configured to detect a body temperature of a user as the first biometric information, based on light of a designated wavelength band, which is received by using the first biometric sensor 705 and 5. The processor 920 may be configured to detect the second biometric information that is different from the first biometric information, using the second biometric sensor 720 and 1020.

In an embodiment, the wearable electronic device 701, 1001, and 1201 may include the second lens 728 and 28. The rear cover 730 and 1030 may have the second seating hole 728h and 28h, in which the second lens 728 and 28 is disposed. The light emitting part 721 and 1021 of the second biometric sensor 720 and 1020 may include the first light emitting part that overlaps the second lens 728 and 28.

In an embodiment, the wearable electronic device 701, 1001, and 1201 may include the third lens 729. The rear cover 730 may have the third seating hole 729h, in which the third lens 729 is disposed. The light emitting part 721 of the second biometric sensor 720 may include the second light emitting part that overlaps the third lens 729. The first lens 709 may be located between the second lens 728 and the third lens 729. The first lens 709, the second lens 728, and the third lens 729 may be at least partially aligned along one direction. The wearable electronic device may include at least one of a first protection layer 820 disposed on a first surface 709A of the first lens 709, which is exposed through the first seating hole 709h, and a second protection layer 830 disposed on a second surface 709B in an opposite direction to the first surface 709A.

In an embodiment, the first lens 709 and 9 may be press-fitted with the first seating hole 709h and 9h.

In an embodiment, the wearable electronic device 701, 1001, and 1201 may include a waterproof adhesion member that is at least partially interposed between the first lens 709 and 9 and an inner peripheral surface of the first seating hole 709h and 9h.

In an embodiment, the first seating hole 9h may include the first hole h1 and the second hole h2 in communication with the first hole h1. The second hole h2 may be located between the first hole h1 and the first biometric sensor 5. The rear cover 1030 may include the seating surface 32C defined as a diameter of the second hole h2 is formed to be smaller than a diameter of the first hole h1. The first lens 9 may be disposed on the seating surface 32C to cover the second hole h2.

In an embodiment, the wearable electronic device 701, 1001, and 1201 may include the second lens 28. The rear cover 1030 may have the second seating hole 28h, in which the second lens 28 is disposed. The biometric sensor module 1000 may include the third sensor 15 that overlaps the second lens 28. The processor 920 may be configured to detect the third biometric information that is different from the first biometric information and the second biometric information, using the third sensor 15.

In an embodiment, the rear cover 730 and 1030 may include sapphire glass. In an embodiment, the first lens 709 and 9 may include a silicon lens.

In an embodiment, the first lens 709 and 9 may include a plurality of fine holes 810, and optic members filled in the plurality of fine holes 810, respectively.

While the disclosure has been illustrated and described with reference to various example embodiments, it will be understood that the various example embodiments are intended to be illustrative, not limiting. It will be further understood by those skilled in the art that various changes in form and detail may be made without departing from the true spirit and full scope of the disclosure, including the appended claims and their equivalents. It will also be understood that any of the embodiment(s) described herein may be used in conjunction with any other embodiment(s) described herein.

## Claims

1. A wearable electronic device (401, 401-1, 401-2, 401-3, 701, 1001, 1201) comprising:
a housing (110) including a rear cover (430, 730, 1030) having a first hole and at least partially contacting a body of a user while the wearable electronic device (401, 401-1, 401-2, 401-3, 701, 1001, 1201) is worn by the user;
a first biometric sensor (400, 705, 5) disposed in the housing (110) and aligned with the first hole (H1, 709h, 9h), wherein the first biometric sensor (400, 705, 5) includes a first lens (409, 709, 9) disposed in the first hole (H1, 709h, 9h) and is configured to detect first biometric information of the user based on light of a designated wavelength band, which passes through the first lens (409, 709, 9);
a second biometric sensor (420, 720, 1020) disposed in the housing (110) and disposed to face the rear cover (430, 730, 1030), wherein the second biometric sensor (420, 720, 1020) is configured to detect second biometric information that is different from the first biometric information; and
a blocking structure (10, 20, 30) configured to prevent or reduce foreign substances from being introduced through the first hole (H1, 709h, 9h).

2. The wearable electronic device of claim 1, wherein the first biometric sensor (400, 705, 5) is configured to detect a temperature as the first biometric information, based on the light of the designated wavelength band, which is input into the first hole (H1, 709h, 9h).

3. The wearable electronic device of claim 1, wherein the rear cover (430) includes a first member (431) and a second member (432) disposed under the first member (431),
wherein the second member (432) is coupled to the first member (431) to close an opening (OP2) formed at a central portion of the first member (431),
wherein the first hole (H1) is formed in the first member (431) to be exposed through a rear surface (110B) of the wearable electronic device (401, 401-1, 401-2, 401-3), and
wherein the second biometric sensor (420) is at least partially disposed in the opening (OP2) to face the second member (432).

4. The wearable electronic device of claim 3, wherein the first biometric sensor (400) includes:
a board (403);
a sensor part (405) disposed in the board (403), wherein the sensor part (405) is disposed to face the first lens (409) to receive the light of the designated wavelength band, which passes through the first lens (409); and
a sensor housing (407) surrounding the sensor part (405), and having a light receiving hole (OP3), to which the first lens (409) is coupled.

5. The wearable electronic device of claim 4, wherein the blocking structure (10, 20, 30) includes a first blocking structure (10),
wherein the sensor housing (407) includes a first part (4071) disposed on the board (403) to surround the sensor part (405), a second part (4072) extending from an inner periphery of the first part (4071) along the sensor part (405), and a third part (4073) extending from the second part (4072) toward the first lens (409), and defining the light receiving hole (OP3), and
wherein the first blocking structure (10) includes a first waterproof member (11) disposed between the first part (4071) and the first member (431), and an adhesion member (12) disposed between the first waterproof member (11) and the first member (431), and
wherein the first waterproof member (11) includes a boss (13) protruding toward the first part (4071) and compressed by the first part (4071).

6. The wearable electronic device of claim 4, wherein the blocking structure (10, 20, 30) includes a second blocking structure (20),
wherein the second blocking structure (20) includes a waterproof adhesion layer (21) interposed between the sensor housing (407) and the first member (431), and
wherein the sensor housing (407) includes a first part (4071) disposed on the board (403) to surround the sensor part (405), a second part (4072) extending from an inner periphery of the first part (4071) along the sensor part (405), and a third part (4073) extending from the second part (4072) toward the first lens (409), and defining the light receiving hole (OP3),
wherein the second part (4072) and the third part (4073) are at least partially inserted into the first hole (H1), and
wherein the waterproof adhesion layer (21) includes:
a first layer (211) interposed between the first part (4071) and the first member (431); and
a second layer (212) interposed between a first inner peripheral surface (S 1) of the first hole (H1) and the second part (4072), and between the first inner peripheral surface (S 1) and the third part (4073).

7. The wearable electronic device of claim 4, wherein the blocking structure (10, 20, 30) includes a third blocking structure (30), and
wherein the sensor housing (407) includes a first part (4071) disposed on the board (403) to surround the sensor part (405), a second part (4072) extending from an inner periphery of the first part (4071) along the sensor part (405), a third part (4073) extending from the second part (4072) toward the first lens (409), and defining the light receiving hole (OP3), and a protrusion (4072a) extending from the second part (4072) to face the first part (4071), and
wherein the third blocking structure (30) includes a second waterproof member (31) interposed between a recess (32) defined by the first part (4071), the second part (4072), and the protrusion (4072a), and the first member (431).

8. The wearable electronic device of claim 1, wherein the first lens (409, 709, 9) includes a first surface (709A) that faces an outside of the wearable electronic device, and a second surface (709B) in an opposite direction to the first surface (709A),
wherein the first lens (709) has a plurality of fine holes (810) that extend from the first surface (709A) to the second surface (709B) and pass through the first lens (709), and
wherein the wearable electronic device includes a first protection layer (820) and/or a second protection layer (830) disposed on the first surface (709A) and/or the second surface (709B) of the first lens (709).

9. The wearable electronic device of any one of claims 1 to 8, wherein the rear cover (430, 730, 1030) includes sapphire glass, and
wherein the first lens (409, 709, 9) includes a silicon lens.

10. An wearable electronic device (701, 1001, 1201) comprising:
a frame (310);
a display (320) disposed on one side of the frame (310);
a rear cover (730, 1030) disposed on an opposite side of the frame (310) and having a first seating hole (709h, 9h);
a printed circuit board (380) disposed between the display (320) and the rear cover (730, 1030);
a processor (920) disposed in the printed circuit board (380);
a biometric sensor module (700, 1000) disposed between the printed circuit board (380) and the rear cover, and including a first biometric sensor (705, 5) and a second biometric sensor (720, 1020) operatively connected to the processor (920), wherein the second biometric sensor (720, 1020) includes a light emitting part (721, 1021) and a light receiving part (723, 1023); and
a first lens (709, 9) disposed in the first seating hole (709h, 9h) and at least partially overlapping the first biometric sensor (705, 5), and
wherein the processor (920) is configured to:
detect a body temperature of a user as first biometric information, based on light of a designated wavelength band, which is received by using the first biometric sensor (705, 5); and
detect second biometric information that is different from the first biometric information, using the second biometric sensor (720, 1020).

11. The wearable electronic device of claim 10, comprising:
a second lens (728, 28),
wherein the rear cover (730, 1030) has a second seating hole (728h, 28h), in which the second lens (728, 28) is disposed, and
wherein the light emitting part (721, 1021) of the second biometric sensor (720, 1020) includes a first light emitting part that overlaps the second lens (728, 28).

12. The wearable electronic device of claim 10, wherein the first lens (709, 9) is press-fitted with the first seating hole (709h, 9h).

13. The wearable electronic device of claim 10, comprising:
a waterproof adhesion member at least partially interposed between the first lens (709, 9) and an inner peripheral surface of the first seating hole (709h, 9h).

14. The wearable electronic device of claim 10, comprising:
a second lens (28),
wherein the rear cover (1030) has a second seating hole (28h), in which the second lens (28) is disposed,
wherein the biometric sensor module (1000) includes a third sensor (15) that overlaps the second lens (28), and
wherein the processor (920) is configured to detect third biometric information that is distinguished from the first biometric information and the second biometric information, by using the third sensor (15).

15. The wearable electronic device of any one of claims 10 to 14, wherein the rear cover (730, 1030) includes sapphire glass, and
wherein the first lens (709, 9) includes a silicon lens.
